# FASCICULE DE BREVET EUROPEEN

(11) **EP 1 862 162 B1**
(45) Date de publication et mention de la délivrance du brevet: **22.06.2016**
(21) Numéro de dépôt: 07107572.5
(22) Date de dépôt: 04.05.2007
(51) Int. Cl.: A61K 8/90, A61K 8/91, A61Q 1/02, A61Q 1/10, A61Q 1/12

(54) **Composition cosmétique comprenant un polymère vinylique et un copolymère d'oléfine**
Kosmetische Zusammensetzung, die ein Vinylpolymer und ein Olefin-Copolymer umfasst
Cosmetic composition comprising a vinyl polymer and an olefin copolymer

(30) Priorité: 31.05.2006 FR 0651988
(43) Date de publication de la demande: 05.12.2007
(73) Titulaire: L'Oréal, 75008 Paris (FR)
(72) Inventeur: Arnaud, Pascal, 94240, L'HAY LES ROSES (FR); Collette, Annick, 94100, ST MAUR DES FOSSES (FR)
(74) Mandataire: Boulard, Denis

(56) Documents cités:
- EP-A- 1 034 776
- WO-A-03/045337
- FR-A- 2 840 204

## Description

La présente invention a pour objet une composition de maquillage ou de soin des matières kératiniques, notamment de la peau, comprenant une phase grasse liquide et au moins un polymère vinylique ayant au moins un motif dérivé de dendrimère carbosiloxane et au moins un copolymère d'oléfine.

Les compositions cosmétiques de maquillage telles que les fonds de teint, les rouges à lèvres, les mascaras, les produits de maquillage du corps, les anticernes, les fards à paupières ou les poudres, comprennent généralement des corps gras tels que des huiles et/ou des cires, et une phase particulaire généralement composée de charges et de pigments. Elles peuvent ainsi se présenter sous la forme d'un gel anhydre, sous forme de stick ou bâton ou sous forme de pâte souple. Elles peuvent encore se présenter sous la forme d'une poudre, qui peut être par exemple libre, compactée ou pressée. Les compositions de maquillage peuvent également comprendre de l'eau ou une phase hydrophile, et se présenter alors notamment sous forme d'émulsion huile-dans-eau, eau-dans-huile, émulsion multiple, notamment lorsqu'il s'agit d'un fond de teint ou de crème teintée.

Les compositions de soin peuvent être en particulier des compositions solaires ou des déodorants.

Les compositions de fond de teint sont couramment employées pour apporter une couleur esthétique à la peau, notamment au visage. Ces produits de maquillage contiennent généralement des huiles, des pigments et/ou charges et éventuellement des additifs comme des actifs cosmétiques ou dermatologiques.

Ces compositions, lorsqu'elles sont appliquées sur la peau, présentent l'inconvénient de transférer, c'est-à-dire de se déposer au moins en partie, en laissant des traces, sur certains supports avec lesquels elles peuvent être mises en contact et notamment un vêtement ou la peau. Il s'ensuit une tenue médiocre du film appliqué, nécessitant de renouveler régulièrement l'application de la composition. Par ailleurs, l'apparition de ces traces inacceptables notamment sur les cols de chemisier peut écarter certaines femmes de l'utilisation de ce type de maquillage.

De plus, le sébum sécrété par la peau au cours du temps modifie également les propriétés du maquillage. En particulier, le sébum ne favorise pas l'adhésion du maquillage sur la peau et le transfert du maquillage est encore plus important, engendrant une perte notable du maquillage restant sur la peau.

L'invention du document FR-A-2 840 204 a pour objet une composition de maquillage de la peau comprenant une phase organique liquide, des particules de polymère dispersées dans la phase organique liquide et stabilisées en surface par un agent stabilisant, des particules de polytétrafluoroéthylène, la phase organique liquide contenant un polymère gélifiant amorphe formé par polymérisation d'une oléfine. Les compositions de ce document présentent des propriétés de non transfert améliorées.

On recherche donc des compositions de maquillage des matières kératiniques, notamment de la peau, qui présentent l'avantage de former un dépôt résistant au transfert, notamment qui ne se dépose pas, au moins en partie, sur les supports avec lesquels elles sont mises en contact (vêtements, tissus).

La présente invention a donc pour but de fournir une nouvelle voie de formulation d'un produit cosmétique qui permette d'obtenir de bonnes propriétés de tenue du maquillage, en particulier de tenue de la couleur et de la matité, ainsi que des propriétés de résistance au transfert.

Or, la Demanderesse a trouvé de manière surprenante qu'en introduisant, dans une composition cosmétique de maquillage ou de soin des matières kératiniques, notamment de la peau, un polymère vinylique ayant au moins un motif dérivé de dendrimère carbosiloxane, et au moins un copolymère d'oléfine, il était possible de réaliser un produit de maquillage présentant une très bonne tenue.

La présente invention a pour objet une composition de maquillage ou de soin des matières kératiniques, en particulier la peau, comprenant une phase grasse liquide comprenant :
i) au moins un polymère vinylique ayant au moins un motif dérivé de dendrimère carbosiloxane présent en une teneur en matière active allant de 1 à 10% en poids par rapport au poids total de la composition et,
ii) au moins un copolymère d'oléfine,
caractérisée en ce que le polymère vinylique greffé avec un dendrimère carbosiloxane est le produit de polymérisation :
(A) de 0,1 à 99 partie en poids d'un ou plusieurs monomère(s) acrylate ou méthacrylate; et
(B) de 100 à 0,1 parties en poids d'un monomère acrylate ou méthacrylate d'un dendrimère carbosiloxane tri[tri(triméthylsiloxy)silyléthyl diméthylsiloxy]silylpropyle répondant à l'une des formules :

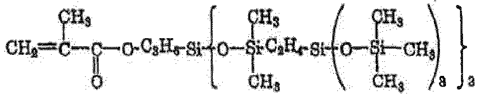

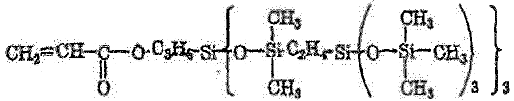

La composition selon l'invention permet d'obtenir un maquillage confortable, sans sensation de collant et s'étalant facilement.

L'invention a également pour objet un procédé non thérapeutique de maquillage ou de soin des matières kératiniques comprenant l'application sur lesdites matières kératiniques d'une composition telle que définie précédemment.

L'invention a encore pour objet l'utilisation d'une composition telle que définie précédemment pour obtenir un maquillage sur les matières kératiniques, présentant une bonne tenue de la couleur et de la matité et résistant au transfert.

### Polymère vinylique greffé avec un dendrimère carbosiloxane

Le polymère vinylique présent dans la composition selon l'invention comprend au moins un motif dérivé de dendrimère carbosiloxane.

Le polymère vinylique peut posséder notamment un squelette et au moins une chaîne latérale, laquelle comprend une structure de dendrimère carbosiloxane. Le terme « structure de dendrimère carbosiloxane » dans le contexte de la présente invention représente une structure moléculaire possédant des groupes ramifiés ayant des masses moléculaires élevées, ladite structure ayant une régularité élevée dans la direction radiale en partant de la liaison au squelette. De telles structures de dendrimère carbosiloxane sont décrites sous la forme d'un copolymère siloxane-silylalkylène fortement ramifié dans la demande de brevet japonais mise à l'inspection publique Kokai 9-171 154.

Le polymère vinylique décrit dans le brevet contient des motifs dérivés de dendrimères carbosiloxane qui peuvent être représentés par la formule générale suivante : 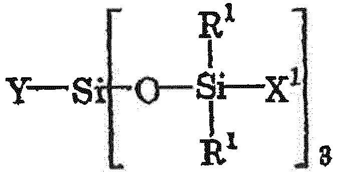 dans laquelle R¹ représente un groupe aryle ou un groupe alkyle possédant de 1 à 10 atomes de carbone, et X¹ représente un groupe silylalkyle qui, lorsque i = 1, est représenté par la formule : 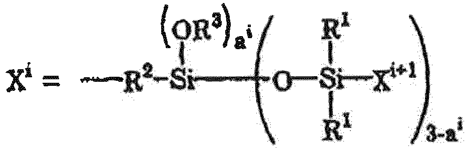 dans laquelle R¹ est le même que défini ci-dessus, R² représente un groupe alkylène possédant de 2 à 10 atomes de carbone, R³ représente un groupe alkyle possédant de 1 à 10 atomes de carbone, Xⁱ⁺¹ représente un atome d'hydrogène, un groupe alkyle possédant de 1 à 10 atomes de carbone, un groupe aryle, ou le groupe silylalkyle défini ci-dessus avec i = i + 1 ; i est un nombre entier de 1 à 10 qui représente la génération dudit groupe silylalkyle, et aⁱ est un nombre entier de 0 à 3 ; Y représente un groupe organique polymérisable à l'aide de radicaux choisi dans le groupe constitué par un groupe organique qui contient un groupe méthacrylique ou un groupe acrylique et qui est représenté par les formules : 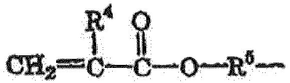 et 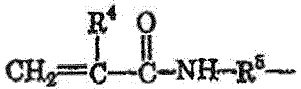 dans lesquelles R⁴ représente un atome d'hydrogène ou un groupe alkyle, R⁵ représente un groupe alkylène possédant de 1 à 10 atomes de carbone tel qu'un groupe méthylène, un groupe éthylène, un groupe propylène, ou un groupe butylène, le groupe méthylène et le groupe propylène étant préférés ; et
un groupe organique qui contient un groupe styryle et qui est représenté par la formule : 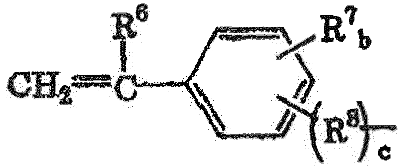 dans laquelle R⁶ représente un atome d'hydrogène ou un groupe alkyle, R⁷ représente un groupe alkyle possédant de 1 à 10 atomes de carbone tel qu'un groupe méthyle, un groupe éthyle, un groupe propyle, ou un groupe butyle, le groupe méthyle étant préféré, R⁸ représente un groupe alkylène possédant de 1 à 10 atomes de carbone tel qu'un groupe méthylène, un groupe éthylène, un groupe propylène, un groupe butylène, le groupe éthylène étant préféré, b est un nombre entier de 0 à 4, et c vaut 0 ou 1 de sorte que si c vaut 0, -(R⁸)_{c}- représente une liaison.
R¹ représente un groupe aryle ou un groupe alkyle possédant de 1 à 10 atomes de carbone, où le groupe alkyle est de préférence représenté par un groupe méthyle, un groupe éthyle, un groupe propyle, un groupe butyle, un groupe pentyle, un groupe isopropyle, un groupe isobutyle, un groupe cyclopentyle, un groupe cyclohexyle, et où le groupe aryle est de préférence représenté par un groupe phényle et un groupe naphtyle, où les groupes méthyle et phényle sont plus particulièrement préférés, et le groupe méthyle est préféré entre tous.

Le polymère vinylique tel que décrit dans le brevet, qui contient une structure de dendrimère carbosiloxane peut être le produit de polymérisation de :
(A) de 0 à 99,9 parties en poids d'un monomère de type vinyle; et
(B) de 100 à 0,1 parties en poids d'un dendrimère carbosiloxane qui contient un groupe organique polymérisable à l'aide de radicaux, représenté par la formule générale : 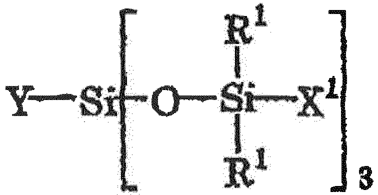 dans laquelle Y représente un groupe organique polymérisable à l'aide de radicaux, R¹ représente un groupe aryle ou un groupe alkyle possédant de 1 à 10 atomes de carbone, et X¹ représente un groupe silylalkyle qui, lorsque i = 1, est représenté par la formule : 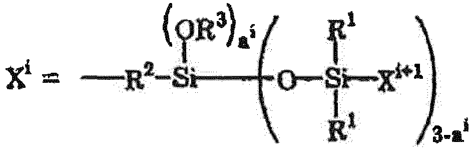 dans laquelle R¹ est le même que défini ci-dessus, R² représente un groupe alkylène possédant de 2 à 10 atomes de carbone, R³ représente un groupe alkyle possédant de 1 à 10 atomes de carbone, Xⁱ⁺¹ représente un atome d'hydrogène, un groupe alkyle possédant de 1 à 10 atomes de carbone, un groupe aryle, ou le groupe silylalkyle défini ci-dessus avec i = i + 1 ; i est un nombre entier de 1 à 10 qui représente la génération dudit groupe silylalkyle, et aⁱ est un nombre entier de 0 à 3 ; où ledit groupe organique polymérisable à l'aide de radicaux contenu dans le composant (B) est choisi dans le groupe constitué par un groupe organique qui contient un groupe méthacrylique ou un groupe acrylique et qui est représenté par les formules : 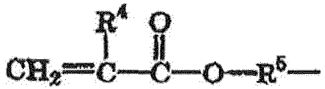 et 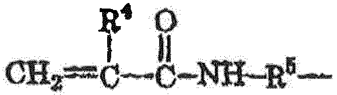 dans lesquelles R⁴ représente un atome d'hydrogène ou un groupe alkyle, R⁵ représente un groupe alkylène possédant de 1 à 10 atomes de carbone ; et
   un groupe organique qui contient un groupe styryle et qui est représenté par la formule : 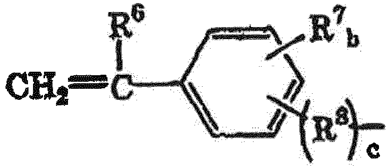 dans laquelle R⁶ représente un atome d'hydrogène ou un groupe alkyle, R⁷ représente un groupe alkyle possédant de 1 à 10 atomes de carbone, R⁸ représente un groupe alkylène possédant de 1 à 10 atomes de carbone, b est un nombre entier de 0 à 4, et c vaut 0 ou 1. Dans le cas où c vaut 0, -(R⁸)_{c}- représente une liaison.

Le monomère de type vinyle qui est le composant (A) dans le polymère vinylique décrit dans le brevet est un monomère de type vinyle qui contient un groupe vinyle polymérisable à l'aide de radicaux. Il n'y a aucune limitation particulière en ce qui concerne le type d'un tel monomère. Ce qui suit sont des exemples de ce monomère de type vinyle : le méthacrylate de méthyle, le méthacrylate d'éthyle, le méthacrylate de n-propyle, le méthacrylate d'isopropyle, ou un méthacrylate d'alkyle analogue inférieur ; le méthacrylate de glycidyle ; le méthacrylate de butyle, l'acrylate de butyle, le méthacrylate de n-butyle, le méthacrylate d'isobutyle, l'acrylate de tert-butyle, le méthacrylate de tert-butyle, le méthacrylate de n-hexyle, le méthacrylate de cyclohexyle, l'acrylate de 2-éthylhexyle, le méthacrylate de 2-éthylhexyle, le méthacrylate d'octyle, le méthacrylate de lauryle, l'acrylate de stéaryle, le méthacrylate de stéaryle, ou un méthacrylate analogue supérieur ; l'acétate de vinyle, le propionate de vinyle, ou un ester de vinyle d'acide gras analogue inférieur ; le caproate de vinyle, le 2-éthylhexoate de vinyle, le laurate de vinyle, le stéarate de vinyle, ou un ester d'acide gras analogue supérieur ; le styrène, le vinyltoluène, le méthacrylate de benzyle, le méthacrylate de phénoxyéthyle, la vinylpyrrolidone, ou des monomères vinyliques aromatiques analogues ; le méthacrylamide, le N-méthylolméthacrylamide, le N-méthoxyméthylméthacrylamide, l'isobutoxyméthoxyméthacrylamide, le N,N-diméthylméthacrylamide, ou des monomères analogues de type vinyle qui contiennent des groupes amide ; le méthacrylate d'hydroxyéthyle, le méthacrylate de l'alcool hydroxypropylique, ou des monomères analogues de type vinyle qui contiennent des groupes hydroxyle ; l'acide acrylique, l'acide méthacrylique, l'acide itaconique, l'acide crotonique, l'acide fumarique, l'acide maléique, ou des monomères analogues de type vinyle qui contiennent un groupe acide carboxylique ; le méthacrylate de tétrahydrofurfuryle, le méthacrylate de butoxyéthyle, le méthacrylate de l'éthoxydiéthylèneglycol, le polyéthylèneglycolméthacrylate, le polypropylèneglycolmonométhacrylate, l'éther d'hydroxybutyle et de vinyle, l'éther de cétyle et de vinyle, l'éther de 2-éthylhexyle et de vinyle, ou un monomère analogue de type vinyle avec des liaisons éther ; le méthacryloxypropyltriméthoxysilane, le polydiméthylsiloxane ayant un groupe méthacrylique sur l'une de ses extrémités moléculaires, le polydiméthylsiloxane ayant un groupe styryle sur une de ses extrémités moléculaires, ou un composé analogue de silicone possédant des groupes insaturés ; le butadiène ; le chlorure de vinyle ; le chlorure de vinylidène ; le méthacrylonitrile ; le dibutylfumarate ; l'acide maléique anhydre ; l'acide succinique anhydre ; l'éther de méthacryle et de glycidyle ; un sel organique d'une aminé, un sel d'ammonium, et un sel de métal alcalin de l'acide méthacrylique, de l'acide itaconique, de l'acide crotonique, de l'acide maléique, ou de l'acide fumarique ; un monomère insaturé polymérisable à l'aide de radicaux possédant un groupe acide sulfonique tel qu'un groupe styrène acide sulfonique ; un sel d'ammonium quaternaire dérivé de l'acide méthacrylique tel que le chlorure de 2-hydroxy-3-méthacryloxypropyltriméthylammonium ; et un ester de l'acide méthacrylique d'un alcool possédant un groupe amine tertiaire tel qu'un ester de l'acide méthacrylique et de la diéthylamine.

Les monomères de type vinyle multifonctionnels peuvent également être utilisés. Ce qui suit représente des exemples de tels composés : le triméthacrylate de triméthylolpropane, le triméthacrylate de pentaérythritol, le diméthacrylate d'éthylèneglycol, le diméthacrylate de tétraéthylèneglycol, le polyéthylèneglycoldiméthacrylate, le diméthacrylate de 1,4-butanediol, le diméthacrylate de 1,6-hexanediol, le diméthacrylate de néopentylglycol, le triméthylolpropanetrioxyéthylméthacrylate, le diméthacrylate de tris-(2-hydroxyéthyl)isocyanurate, le triméthacrylate de tris-(2-hydroxyéthyl)isocyanurate, le polydiméthylsiloxane coiffé de groupes styryle possédant des groupes divinylbenzène sur les deux extrémités, ou des composés analogues de silicone possédant des groupes insaturés.

Le dendrimère carbosiloxane, lequel est le composant (B) décrit dans le brevet, est représenté par la formule suivante :

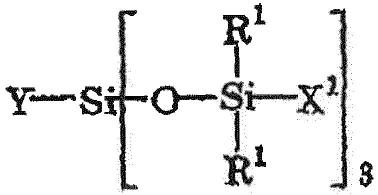

Ce qui suit représente les exemples préférés de groupe organique Y polymérisable à l'aide de radicaux : un groupe acryloxyméthyle, un groupe 3-acryloxypropyle, un groupe méthacryloxyméthyle, un groupe 3-méthacryloxypropyle, un groupe 4-vinylphényle, un groupe 3-vinylphényle, un groupe 4-(2-propényl)phényle, un groupe 3-(2-propényl)phényle, un groupe 2-(4-vinylphényl)éthyle, un groupe 2-(3-vinylphényl)éthyle, un groupe vinyle, un groupe allyle, un groupe méthallyle, et un groupe 5-hexényle.

R¹ représente un groupe alkyle ou un groupe aryle possédant de 1 à 10 atomes de carbone, où le groupe alkyle peut être un groupe méthyle, un groupe éthyle, un groupe propyle, un groupe butyle, un groupe pentyle, un groupe isopropyle, un groupe isobutyle, un groupe cyclopentyle, ou un groupe cyclohexyle ; et le groupe aryle peut être un groupe phényle ou un groupe naphtyle. Les groupes méthyle et phényle sont particulièrement préférés, le groupe méthyle étant préféré entre tous. X¹ représente un groupe silylalkyle qui est représenté par la formule suivante, lorsque i est égal à un : 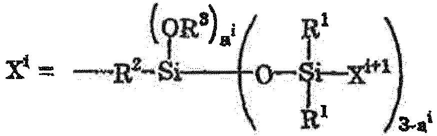 dans laquelle R² représente un groupe alkylène possédant de 2 à 10 atomes de carbone, tel qu'un groupe éthylène, un groupe propylène, un groupe butylène, un groupe hexylène, ou un groupe alkylène linéaire analogue ; un groupe méthylméthylène, un groupe méthyléthylène, un groupe 1-méthylpentylène, un groupe 1,4-diméthylbutylène, ou un groupe alkylène ramifié analogue. Les groupes éthylène, méthyléthylène, hexylène, 1-méthylpentylène et 1,4-diméthylbutylène sont préférés entre tous. R³ représente un groupe alkyle possédant de 1 à 10 atomes de carbone, tel que les groupes méthyle, éthyle, propyle, butyle, et isopropyle. R¹ est le même que défini ci-dessus. Xⁱ⁺¹ représente un atome d'hydrogène, un groupe alkyle possédant de 1 à 10 atomes de carbone, un groupe aryle, ou le groupe silylalkyle avec i = i + 1. aⁱ est un nombre entier de 0 à 3, et i est un nombre entier de 1 à 10 qui indique le nombre de génération qui représente le nombre de répétitions du groupe silylalkyle.

Par exemple, lorsque le nombre de génération est égal à un, le dendrimère carbosiloxane peut être représenté par la première formule générale montrée ci-dessous, dans laquelle Y, R¹, R² et R³ sont les mêmes que définis ci-dessus, R¹² représente un atome d'hydrogène ou est identique à R¹ ; a¹ est identique à aⁱ, De préférence, le nombre moyen total de groupes OR³ dans une molécule est dans la plage de 0 à 7. Lorsque le nombre de génération est égal à 2, le dendrimère carbosiloxane peut être représenté par la deuxième formule générale montrée ci-dessous, dans laquelle Y, R¹, R², R³ et R¹² sont les mêmes que définis ci-dessus ; a¹ et a² représentent le aⁱ de la génération indiquée. De préférence, le nombre moyen total de groupes OR³ dans une molécule est dans la plage de 0 à 25. Dans le cas où le nombre de génération est égal à 3, le dendrimère carbosiloxane est représenté par la troisième formule générale montrée ci-dessous, dans laquelle Y, R¹, R², R³ et R¹² sont les mêmes que définis ci-dessus; a¹, a² et a³ représentent le aⁱ de la génération indiquée. De préférence, le nombre moyen total de groupes OR³ dans une molécule est dans la plage de 0 à 79.

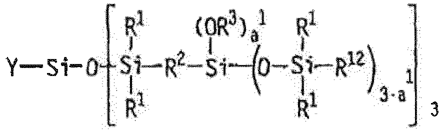

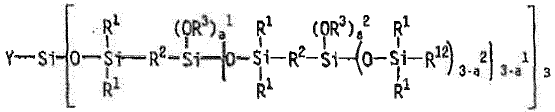

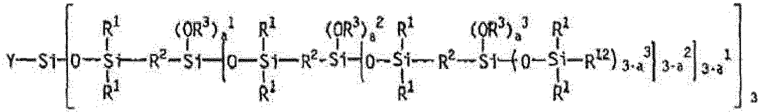

Un dendrimère carbosiloxane qui contient un groupe organique polymérisable à l'aide de radicaux peut être représenté par les formules de structures moyennes suivantes :

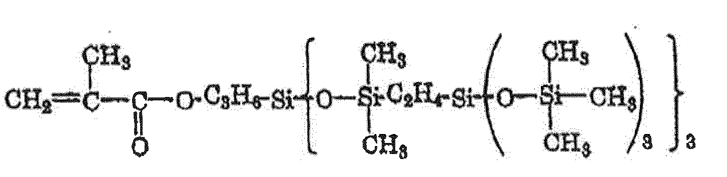

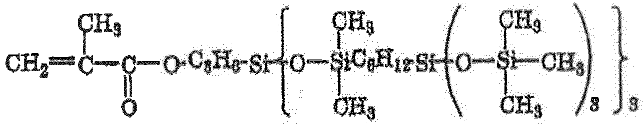

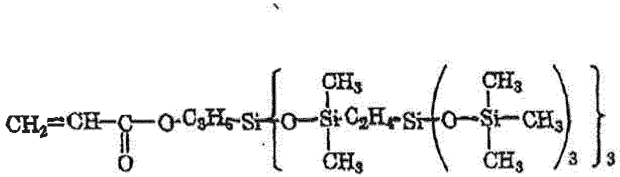

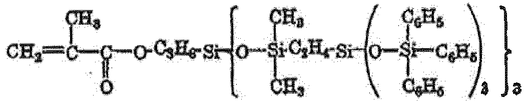

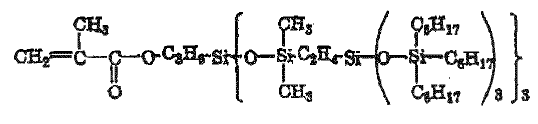

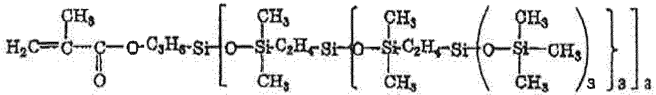

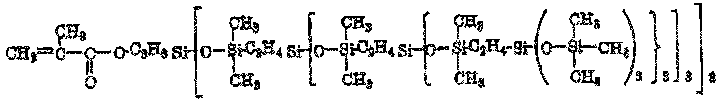

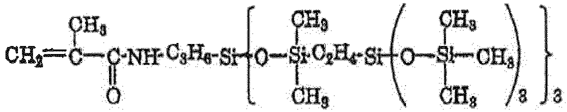

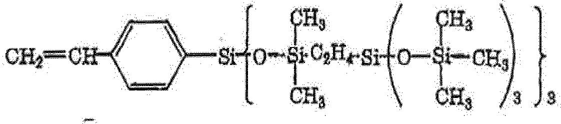

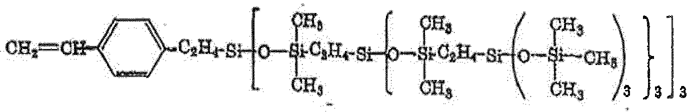

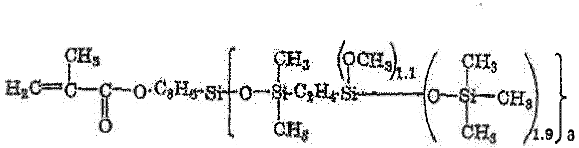

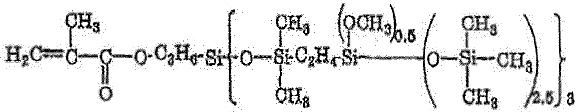

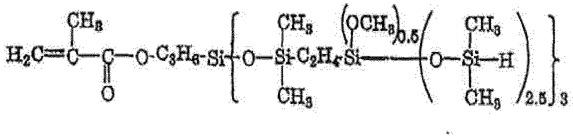

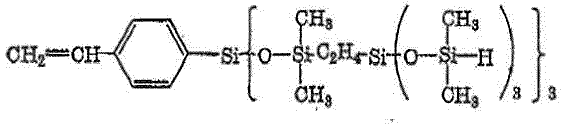

Le dendrimère carbosiloxane décrit dans le brevet peut être fabriqué selon le procédé pour fabriquer un siloxane silalkylène ramifié décrit dans la demande de brevet japonais Hei 9-171 154. Par exemple, il peut être produit en soumettant à une réaction d'hydrosilylation un composé organosilicium qui contient un atome d'hydrogène relié à un atome de silicium, représenté par la formule générale suivante : 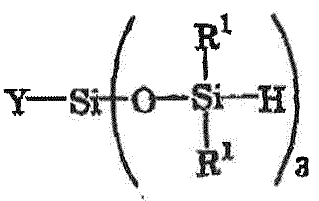 et un composé organosilicium qui contient un groupe alcényle. Dans la formule ci-dessus, le composé organosilicium peut être représenté par le 3-méthacryloxypropyltris-(diméthylsiloxy)silane, le 3-acryloxypropyltris-(diméthylsiloxy)silane, et le 4-vinylphényltris-(diméthylsiloxy)silane. Le composé organosilicium qui contient un groupe alcényle peut être représenté par le vinyltris-(triméthylsiloxy)silane, le vinyltris-(diméthylphénylsiloxy)silane, et le 5-hexényltris-(triméthylsiloxy)silane. La réaction d'hydrosilylation est réalisée en présence d'un acide chloroplatinique, d'un complexe de vinylsiloxane et de platine, ou d'un catalyseur analogue d'un métal de transition.

Dans le polymère vinylique qui contient une structure de dendrimère tel que décrit dans le brevet, le rapport de polymérisation entre les composants (A) et (B), en termes de rapport en poids entre (A) et (B), peut être dans une plage de 0/100 à 99,9/0,1, et de préférence dans une plage de 1/99 à 99/1. Un rapport entre les composants (A) et (B) de 0/100 signifie que le composé devient un homopolymère de composant (B).

Le polymère vinylique tel que décrit dans le brevet contient une structure de dendrimère carbosiloxane et ce polymère peut être obtenu par la copolymérisation des composants (A) et (B), ou par la polymérisation du seul composant (B). La polymérisation peut être une polymérisation radicalaire ou une polymérisation ionique, toutefois la polymérisation radicalaire est préférée. La polymérisation peut être réalisée en provoquant une réaction entre les composants (A) et (B) dans une solution pendant une période de 3 à 20 heures en présence d'un initiateur de radicaux à une température de 50°C à 150°C. Un solvant approprié dans ce but est l'hexane, l'octane, le décane, le cyclohexane, ou un hydrocarbure aliphatique analogue ; le benzène, le toluène, le xylène, ou un hydrocarbure aromatique analogue ; l'éther diéthylique, l'éther dibutylique, le tétrahydrofurane, le dioxane, ou des éthers analogues ; l'acétone, la méthyléthylcétone, la méthylisobutylcétone, la di-isobutylcétone, ou des cétones analogues ; l'acétate de méthyle, l'acétate d'éthyle, l'acétate de butyle, l'acétate d'isobutyle, ou des esters analogues ; le méthanol, l'éthanol, l'isopropanol, le butanol, ou des alcools analogues ; l'octaméthylcyclotétrasiloxane, le décaméthylcyclopentasiloxane, l'hexaméthyldisiloxane, l'octaméthyltrisiloxane, ou un oligomère organosiloxane analogue. Un initiateur de radicaux peut être tout composé connu dans l'art pour des réactions classiques de polymérisation radicalaire. Les exemples spécifiques de tels initiateurs de radicaux sont le 2,2'-azobis(isobutyronitrile), le 2,2'-azobis(2-méthylbutyronitrile), le 2,2'-azobis(2,4-diméthylvaléronitrile) ou des composés analogues de type azobis ; le peroxyde de benzoyle, le peroxyde de lauroyle, le peroxybenzoate de tert-butyle, le peroxy-2-éthylhexanoate de tert-butyle, ou un peroxyde organique analogue. Ces initiateurs de radicaux peuvent être utilisés seuls ou dans une combinaison de deux ou plus. Les initiateurs de radicaux peuvent être utilisés dans une quantité de 0,1 à 5 parties en poids pour 100 parties en poids des composants (A) et (B). Un agent de transfert de chaîne peut être ajouté. L'agent de transfert à chaîne peut être le 2-mercaptoéthanol, le butylmercaptan, le n-dodécylmercaptan, le 3-mercaptopropyltriméthoxysilane, un polydiméthylsiloxane possédant un groupe mercaptopropyle ou un composé analogue de type mercapto ; le chlorure de méthylène, le chloroforme, le tétrachlorure de carbone, le bromure de butyle, le 3-chloropropyltriméthoxysilane, ou un composé halogéné analogue. Dans la fabrication du polymère de type vinyle, après la polymérisation, le monomère vinylique résiduel qui n'a pas réagi peut être éliminé dans des conditions de chauffage sous vide.

Pour faciliter la préparation de mélange de la matière première de produits cosmétiques, la masse moléculaire moyenne en nombre du polymère vinylique qui contient un dendrimère carbosiloxane, peut être choisie dans la plage entre 3 000 et 2 000 000, de préférence entre 5 000 et 800 000. Il peut être un liquide, une gomme, une pâte, un solide, une poudre, ou toute autre forme. Les formes préférées sont les solutions constituées par la dilution dans des solvants, d'une dispersion, ou d'une poudre.

Le polymère vinylique peut être une dispersion d'un polymère de type vinyle ayant une structure de dendrimère carbosiloxane dans sa chaîne moléculaire latérale, dans un liquide tel qu'une huile de silicone, une huile organique, un alcool, ou l'eau.

Le polymère vinylique ayant une structure de dendrimère carbosiloxane dans sa chaîne moléculaire latérale, dans ce mode de réalisation, est le même que celui décrit ci-dessus. Le liquide peut être une huile de silicone, une huile organique, un alcool, ou l'eau. L'huile de silicone peut être un diméthylpolysiloxane ayant les deux extrémités moléculaires coiffées de groupes triméthylsiloxy, un copolymère de méthylphénylsiloxane et de diméthylsiloxane ayant les deux extrémités moléculaires coiffées de groupes triméthylsiloxy, un copolymère de méthyl-3,3,3-trifluoropropylsiloxane et de diméthylsiloxane ayant les deux extrémités moléculaires coiffées de groupes triméthylsiloxy, ou des huiles de silicone linéaires non-réactives analogues, aussi bien que l'hexaméthylcyclotrisiloxane, l'octaméthylcyclotétrasiloxane, le décaméthylcyclopentasiloxane, le dodécaméthylcyclohexasiloxane, ou un composé cyclique analogue. En plus des huiles de silicone non-réactives, des polysiloxanes modifiés possédant des groupes fonctionnels tels que des groupes silanol, des groupes amino, et des groupes polyéther sur les extrémités ou à l'intérieur des chaînes moléculaires latérales peuvent être utilisés.

Les huiles organiques peuvent être l'huile de paraffine, l'isoparaffine, le laurate d'hexyle, le myristate d'isopropyle, le myristate de myristyle, le myristate de cétyle, le myristate de 2-octyldodécyle ; le palmitate d'isopropyle, le palmitate de 2-éthylhexyle, le stéarate de butyle, l'oléate de décyle, l'oléate de 2-octyldodécyle, le lactate de myristyle, le lactate de cétyle, l'acétate de lanoline, l'alcool stéarique, l'alcool cétostéarique, l'alcool oléique, l'huile d'avocat, l'huile d'amande, l'huile d'olive, l'huile de cacao, l'huile de jojoba, l'huile de gomme, l'huile de tournesol, l'huile de soja, l'huile de camélia, le squalane, l'huile de ricin, l'huile de vison, l'huile de graine de coton, l'huile de noix de coco, l'huile de jaune d'oeuf, le suif de boeuf, le saindoux, le monooléate de polypropylèneglycol, le 2-éthylhexanoate de néopentylglycol, ou une huile d'ester de glycol analogue ; l'isostéarate de triglycéryle, le triglycéride d'un acide gras d'huile de noix de coco, ou une huile d'ester d'alcool polyhydrique analogue ; l'éther de polyoxyéthylène et de lauryle, l'éther de polyoxypropylène et de cétyle, ou un éther de polyoxyalkylène analogue.

L'alcool peut être de n'importe quel type approprié pour une utilisation conjointement avec une matière première de produits cosmétiques. Par exemple, il peut être le méthanol, l'éthanol, le butanol, l'isopropanol ou des alcools analogues inférieurs. Une solution ou une dispersion de l'alcool devrait avoir une viscosité dans la plage de 10 à 10⁹ mPa à 25°C. Pour améliorer les propriétés de sensation d'utilisation dans un produit cosmétique, la viscosité devrait être dans la plage de 100 à 5 x 10⁸ mPa.s.

Les solutions et les dispersions peuvent facilement être préparées en mélangeant le polymère vinylique ayant une structure de dendrimère carbosiloxane tel que décrit dans le brevet, à une huile de silicone, une huile organique, un alcool, ou de l'eau. Les liquides peuvent être présents dans l'étape de polymérisation du polymère de type vinyle ayant une structure de dendrimère carbosiloxane. Dans ce cas, le monomère vinylique résiduel qui n'a pas réagi devrait être complètement éliminé par traitement thermique de la solution ou de la dispersion sous pression atmosphérique ou réduite. Dans le cas d'une dispersion, la dispersité du polymère de type vinyle peut être améliorée en ajoutant un agent tensio-actif. Un tel agent peut être l'acide hexylbenzènesulfonique, l'acide octylbenzènesulfonique, l'acide décylbenzènesulfonique, l'acide dodécylbenzènesulfonique, l'acide cétylbenzènesulfonique, l'acide myristylbenzènesulfonique, ou des agents tensio-actifs anioniques des sels de sodium de ces acides ; l'hydroxyde d'octyltriméthylammonium, l'hydroxyde de dodécyltriméthylammonium, l'hydroxyde d'hexadécyltriméthylammonium, l'hydroxyde d'octyldiméthylbenzylammonium, l'hydroxyde de décyldiméthylbenzylammonium, l'hydroxyde de dioctadécyldiméthylammonium, l'hydroxyde de suif de boeuf-triméthylammonium, l'hydroxyde d'huile de noix de coco-triméthylammonium, ou un agent tensio-actif cationique analogue ; un éther d'alkyle de polyoxyalkylène, un polyoxyalkylènealkylphénol, un ester d'alkyle de polyoxyalkylène, l'ester de sorbitol de polyoxyalkylène, le polyéthylèneglycol, le polypropylèneglycol, un additif de l'oxyde d'éthylène de diéthylèneglycol triméthylnonanol, et des agents tensio-actifs non ioniques de type polyester, aussi bien que des mélanges. En outre, les solvants et les dispersions peuvent être combinés avec de l'oxyde de fer approprié pour une utilisation avec les produits cosmétiques, ou un pigment analogue, aussi bien que de l'oxyde de zinc, de l'oxyde de titane, de l'oxyde de silicium, du mica, du talc ou des oxydes inorganiques analogues sous forme de poudre. Dans la dispersion, un diamètre moyen des particules de polymère de type vinyle peut être dans une plage comprise entre 0,001 et 100 microns, de préférence entre 0,01 et 50 microns. En effet, au-delà de la plage recommandée, un produit cosmétique mélangé à l'émulsion n'aura pas une sensation suffisamment bonne sur la peau ou au toucher, ni des propriétés d'étalement suffisantes ni une sensation plaisante.

Le polymère vinylique contenu dans la dispersion ou la solution peut avoir une concentration dans une plage comprise entre 0,1 et 95 % en poids, de préférence entre 5 et 85 % en poids. Cependant, pour faciliter la manipulation et la préparation de mélange, la plage devrait être de préférence entre 10 et 75 % en poids.

Le polymère vinylique décrit dans le brevet peut être un des polymères décrits dans les exemples de la demande EP0963751.

Selon l'invention, le polymère vinylique greffé avec un dendrimère carbosiloxane est le produit de polymérisation :
(A) de 0,1 à 99 partie en poids d'un ou plusieurs monomère(s) acrylate ou méthacrylate; et
(B) de 100 à 0,1 parties en poids d'un monomère acrylate ou méthacrylate d'un dendrimère carbosiloxane tri[tri(triméthylsiloxy)silyléthyl diméthylsiloxy]silylpropyle répondant à l'une des formules :

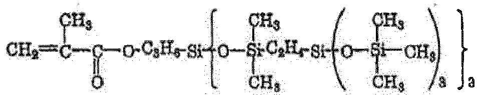

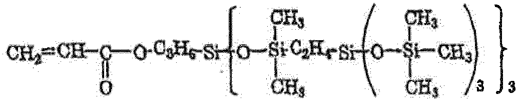

Selon un mode préféré, le polymère vinylique greffé avec un dendrimère carbosiloxane utilisé comprend au moins un monomère acrylate de butyle.

Selon un mode de réalisation, le polymère vinylique comprend en outre au moins un groupement organique fluoré.

On préfère particulièrement des structures dans lesquelles les motifs polymérisés vinyliques constituent le squelette et des structures dendritiques carbosiloxane ainsi que des groupements organiques fluorés sont fixés sur des chaînes latérales.
Les groupements organiques fluorés peuvent être obtenus en substituant par des atomes de fluor tout ou partie des atomes d'hydrogène de groupements méthyle, éthyle, propyle, isopropyle, butyle, isobutyle, tert-butyle, pentyle, néopentyle, hexyle, cyclohexyle, heptyle, octyle, nonyle, décyle, undécyle, dodécyle, tridécyle, tétradécyle, hexadécyle, octadécyle, et d'autres groupements alkyle de 1 à 20 atomes de carbone, ainsi que des groupements alkyloxyalkylène de 6 à 22 atomes de carbone.
Les groupements représentés par la formule : -(CH₂)ₓ-(CF₂)_{y}-R¹³ sont suggérés à titre d'exemple de groupements fluoroalkyle, obtenus en substituant des atomes de fluor pour des atomes d'hydrogène de groupements alkyle. Dans la formule, l'indice « x » et 0, 1, 2 ou 3 et « y » et un entier de 1 à 20. R¹³ est un atome ou un groupement choisi parmi un atome d'hydrogène, un atome de fluor, -(CH(CF₃)₂- ou CF(CF₃)₂. De tels groupements alkyle substitués par fluor sont exemplifiés par des groupements polyfluoroalkyle ou perfluoroalkyle linéaires ou ramifiés représentés par les formules présentées ci-dessous :
- CF₃, -C₂F₅, -nC₃F₇, -CF(CF₃)₂, -nC₄F₉, CF₂CF(CF₃)₂, -nC₅F₁₁, -nC₆F₁₃, -nC₈F₁₇,-CH₂CF₃, -(CH(CF₃)₂, CH₂CH(CF₃)₂-CH₂(CF₂)₂F, -CH₂(CF₂)₃F, -CH₂(CF₂)₄F, -CH₂(CF₂)₆F, -CH₂(CF₂)₈F, -CH₂CH₂CF₃, -CH₂CH₂(CF₂)₂F, -CH₂CH₂(CF₂)₃F, -CH₂CH₂(CF₂)₄F,-CH₂CH₂(CF₂)₆F, -CH₂CH₂(CF₂)₈F, -CH₂CH₂(CF₂)₁₀F,-CH₂CH₂(CF₂)₁₂F, -CH₂CH₂(CF₂)₁₄F, -CH₂CH₂(CF₂)₁₆F,-CH₂CH₂CH₂CF₃, -CH₂CH₂CH₂(CF₂)₂F, -CH₂CH₂CH₂(CF₂)₂H -CH₂(CF₂)₄H, et -CH₂CH₂(CF₂)₃H.
Les groupements représentés par :
- CH₂CH₂-(CF₂)ₘ-CFR¹⁴-[OCF₂CF(CF₃)]ₙ-OC₃F₇ sont suggérés comme des groupements fluoroalkyloxyfluoroalkylène obtenus en substituant des atomes de fluor pour des atomes d'hydrogène des groupements alkyoxyalkylène. Dans la formule, l'indice « m » est 0 ou 1, « n » est 0, 1, 2, 3, 4, ou 5, et R¹⁴ est un atome de fluor ou CF₃. De tels groupements fluoroalkyloxyfluoroalkylène sont exemplifiés par les groupements perfluoroalkyloxyfluoroalkylène représentés par les formules présentées ci-dessous. -CH₂CH₂CF(CF₃)-[OCF₂CF(CF₃)]ₙ-OC₃F₇, -CH₂CH₂CF₂CF₂-[OCF₂CF(CF₃)]ₙ-OC₃F₇.
Le poids moléculaire moyen en nombre du polymère vinylique utilisé dans la présente invention peut être compris entre 3000 et 2 000 000, et, plus préférablement entre 5000 et 800000.
Ce type de polymère vinylique fluoré peut être obtenu par addition :
- d'un monomère vinylique (B) qui n'a pas de groupement organique fluoré dans la molécule
- sur un monomère vinylique contenant des groupements organiques fluorés dans la molécule (A), et
- un dendrimère carbosiloxane (C) contenant des groupements organiques polymérisables par voie radicalaire représenté par la formule générale (III) : 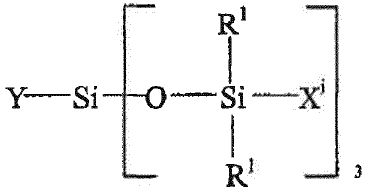 dans laquelle Y est un groupement organique polymérisable par voie radicalaire et R¹ et Xⁱ sont tels que ci-dessus et en les soumettant à une copolymérisation.
Les monomères vinyliques (A) contenant des groupements organiques fluorés dans la molécule sont de préférence des monomères représentés par la formule générale :-
(CH₂)=CR¹⁵COOR^{f}. Dans la formule, R¹⁵ est un atome d'hydrogène ou un groupement méthyle, R^{f} est un groupement organique fluoré exemplifié par les groupements fluoroalkyle et fluoroalkyloxyfluoroalkylène décrits ci-dessus. Les composés représentés par les formules présentées ci-dessous sont suggérés à titre d'exemples spécifiques du composant (A). Dans les formules présentes ci-dessous, « z » est un entier de 1 à 4. CH₂=CCH₃COO-CF₃. CH₂=CCH₃COO-C₂F₅. CH₂=CCH₃COO- nC₃F₇. CH₂=CCH₃COO-CF (CF₃)₂. CH₂=CCH₃COO-nC₄F₉. CH₂=CCH₃COO-CF ₂ CF(CF₃)_{2,} CH₂=CCH₃COO-nC₅F₁₁, CH₂=CCH₃COO-nC₆F₁₃, CH₂=CCH₃COO-nC₈F₁₇, CH₂=CCH₃COO-CH₂CF₃, CH₂-CCH₃COO-CH(CF₃)₂. CH₂=OCH₃COO-CH₂CH(CF₃)₂, CH₂=CCH₃COO-CH₂ (CF₂)₂F, CH₂=CCH₃COO-CH₂(CF₂)₃F, CH₂=CCH₃COO-CH₂(CF₂)₄F, CH₂=CCH₃COO-CH₂(CF₂)₆F, CH₂=CCH₃COO-CH₂ (CF₂)₈F. CH₂=CCH₃COO-CH₂CH₂CF₃, CH₂=CCH₃COO-CH₂CH₂ (CF₂)₂F. CH₂=CCH₃COO-CH₂CH₂(CF₂)₃F, CH₂=CCH₃COO-CH₂CH₂(CF₂)₄F, CH₂=CCH₃COO-CH₂CH₂(CF₂)₆F, CH₂=CCH₃COO-CH₂CH₂(CF₂)₈F, CH₂₌CH₃COO-CH₂CH2(CF₂)₁₀F, CH₂=CCH₃COO-CH₂CH₂(CF₂)₁₂F, CH₂=CCH₃COO-CH₂CH₂(CF₂)₁₄F, CH₂=CCH₃COO-CH₂CH₂(CF₂)₁₆F, CH₂₌CCH₃COOCH₂CH₂CH₂CF₃, CH₂=CCH₃COO-CH₂CH₂CH₂(CF₂)₂F, CH₂=CCH₃COO-CH₂CH₂CH₂ (CF₂)₂H, CH₂=CCH₃COO-CH₂(CF₂)₄H, CH₂=CCH₃COO-CH₂CH₂(CF₂)₃H, CH₂=CCH₃COO-CH₂CH₂CF(CF₃)-[OCF₂CF(CF₃)]_{Z}-OC₃F₇, CH₂=CCH₃COO-CH₂CH₂CF₂CF₂-[OCF₂CF(CF₃)]_{Z}-OC₃F₇, CH₂=CHCOO-CF₃. CH₂=CHCOO-C₂F₅, CH₂=CHCOO-nC₃F₇, CH₂=CHCOO-CF(CF₃)₂, CH₂=CHCOO-nC₄F₉. CH₂=HCOO-CF₂CF(CF₃)₂, CH₂=CHCOO-nC₅F₁₁, CH₂₌CHCOO-nC₆F₁₃, CH₂=CHCOO-nC₈F₁₇, CH₂=CHCOO-CH₂CF₃, CH₂=CHCOO-CH(CF₃)₂, CH₂=CHCOO-CH₂CH(CF₃)₂, CH₂=CHCOO-CH₂(CF₂)₂F, CH₂=CHCOO-CH₂(CF₂)₃F, CH₂=CHCOO-CH₂(CF₂)₄F, CH₂=CHCOO-CH₂(CF₂)₆F, CH₂=CHCOO-CH₂(CF₂)₈F, CH₂=CHCOO-CH₂CH₂CF₃, CH₂=CHCOO-CH₂CH₂(CF₂)₂F, CH₂=CHCOO-CH₂CH₂(CF₂)₃F, CH₂=CHCOO-CH₂CH₂(CF₂)₄F, CH₂=CHCOO-CH₂CH₂(CF₂)₆F, CH₂=CHCOO-CH₂CH₂(CF₂)₈F, CH₂=CHCOO-CH₂CH₂(CF₂)₁₀F, CH₂=CHCOO-CH₂CH₂(CF₂)₁₂F, CH₂=CHCOO-CH₂CH₂(CF₂)₁₄F, CH₂=CHCOO-CH₂CH₂(CF₂)₁₆F, CH₂=CHCOO-CH₂CH₂CH₂CF₃, CH₂=CHCOO-CH₂CH₂CH₂(CF₂)₂F, CH₂=CHCOO-CH₂CH₂CH₂(CF)₂H, CH₂=CHCOO-CH₂(CF₂)₄H, CH₂=CHCOO-CH₂CH₂(CF₂)₃H, CH₂=CHCOO-CH₂CH₂CF(CF₃)-[OCF₂CF(CF₃)]_{Z}-OC₃F₇. CH₂=CHCOO-CH₂CH₂CF₂CF₂₋[OCF₂CF(CF₃)]_{Z}-OC₃F₇.

Parmi ceux-ci, les polymères vinyliques représentés par les formules présentées ci-dessous sont préférables :
CH₂=CHCOO-CH₂CH₂(CF₂)₆F, CH₂=CHCOO-CH₂CH₂(CF₂)₈F, CH₂=CCH₃COO-CH₂CH₂(CF₂)₆F, CH₂=CCH₃COO-CH₂CH₂(CF₂)₈F, CH₂=CHCOO-CH₂CF₃, CH₂-CCH₃COO-CH₂CF₃

Les polymères vinyliques représentés par les formules présentées ci-dessous sont particulièrement préférables :
CH₂=CHCOO-CH₂CF₃, CH₂=CCH₃COO-CH₂CF₃.

Les monomères vinyliques (B) tels que décrits dans le brevet qui ne contiennent pas de groupements organiques fluorés dans la molécule peuvent être des monomères quelconques ayant des groupements vinyliques polymérisables par voie radicalaire qui sont exemplifiés par exemple par l'acrylate de méthyle, le méthacrylate de méthyle, l'acrylate d'éthyle, le méthacrylate d'éthyle, l'acrylate de n-propyle, le méthacrylate de n-propyle, l'acrylate d'isopropyle, le méthacrylate d'isopropyle, et d'autres acrylates ou méthacrylates d'alkyles inférieurs ; l'acrylate de glycidyle, le méthacrylate de glycidyle ; l'acrylate de n-butyle, le méthacrylate de n-butyle, l'acrylate d'isobutyle, le méthacrylate d'isobutyle, l'acrylate de tert-butyle, le méthacrylate de tert-butyle, l'acrylate de n-hexyle, le méthacrylate de n-hexyle, l'acrylate de n-hexyle, le méthacrylate de n-hexyle, l'acrylate de cyclohexyle, le méthacrylate de cyclohexyle, l'acrylate de 2-éthylhexyle, le méthacrylate de 2-éthylhexyle, l'acrylate d'octyle, le méthacrylate d'octyle, l'acrylate de lauryle, le méthacrylate de lauryle, l'acrylate de stéaryle, le méthacrylate de stéaryle, et d'autres acrylates et méthacrylates supérieurs ; l'acétate de vinyle, le propionate de vinyle, et d'autres esters vinyliques d'acide gras inférieurs ; le butyrate de vinyle, le caproate de vinyle, le 2-éthylhexanoate de vinyle, le laurate de vinyle, le stéarate de vinyle, et d'autres esters d'acide gras supérieurs ; le styrène, le vinyltoluène, l'acrylate de benzyle, le méthacrylate de benzyle, l'acrylate de phénoxyéthyle, le méthacrylate de phénoxyéthyle, la vinylpyrrolidone, et d'autres monomères vinyliques aromatiques ; l'acrylate de diméthylaminoéthyle, le méthacrylate de diméthylaminoéthyle, l'acrylate de diéthylaminoéthyle, le méthacrylate de diéthylaminoéthyle, et d'autres monomères vinyliques à amino, l'acrylamide, le méthacrylamide, le N-méthylolacrylamide, le N-méthylolméthacrylamide, le N-méthoxyméthylacrylamide, le N-méthoxyméthyméthacrylamide, l'isobutoxyméthoxyacrylamide, l'isobutoxyméthoxyméthacrylamide, le N,N-diméthylacrylamide, le N,N-diméthylméthacrylamide ,et d'autres monomères vinyliques à amides ; l'acrylate d'hydroxyéthyle, le méthacrylate d'hydroxyéthyle, l'alcool hydroxypropylique d'acide acrylique, l'alcool hydroxypropylique d'acide méthacrylique et d'autres monomères vinyliques à hydroxy ; l'acide acrylique, l'acide méthacrylique, l'acide itaconique, l'acide crotonique, l'acide fumarique, l'acide maléique, et d'autres monomères vinyliques à acide carboxylique ; l'acrylate de tétrahydrofurfuryle, le méthacrylate de tétrahydrofurfuryle, l'acrylate de butoxyéthyle, le méthacrylate de butoxyéthyle, l'acrylate d'éthoxydiéthylèneglycol, le méthacrylate d'éthoxydiéthylèneglycol, l'acrylate de polyéthylèneglycol, le méthacrylate de polyéthylèneglycol, le monoacrylate de polypropylèneglycol, le monométhacrylate de polypropylèneglycol, l'hydroxybutylvinyléther, le cétylvinyléther, le 2-éthylhexylvinyléther et d'autres monomères vinyliques à liaisons éther ; l'acryloxypropyltriméthoxysilane, le méthacryloxypropyltriméthoxysilane, les polydiméthylsiloxanes contenant des groupements acryle ou méthacryle à l'une des extrémités, les polydiméthylsiloxanes contenant des groupements alcénylaryle à l'une des extrémités et d'autres composés siliconés à groupements insaturés ; le butadiène ; le chlorure de vinyle ; le chlorure de vinylidène, l'acrylonitrile, le méthacrylonitrile ; le fumarate de dibutyle ; l'anhydride maléique ; l'anhydride dodécylsuccinique ; l'acrylglycidyléther, le méthacrylglycidyléther, l'acrylate de 3,4-époxycyclohexylméthyle, le méthacrylate de 3,4-époxycyclohexylméthyle, les sels de métaux alcalins, les sels d'ammonium et les sels d'amines organiques d'acide acrylique, d'acide méthacrylique, d'acide itaconique, d'acide crotonique, d'acide fumarique, d'acide maléique et d'autres acides carboxyliques insaturés polymérisables par voie radicalaire, les monomères insaturés polymérisables par voie radicalaire contenant des groupements acide sulfonique tels que l'acide styrènesulfonique ainsi que leurs sels de métaux alcalins, leurs sels d'ammonium et leurs sels d'amine organique ; les sels d'ammonium quaternaire issus d'acide acrylique ou d'acide méthacrylique tels que le chlorure de 2-hydroxy-3-méthacryloxypropyltriméthylammonium, les esters d'acide méthacrylique d'un alcool à aminé tertiaire tels que l'ester de diéthylamine d'acide méthacrylique et leurs sels d'ammonium quaternaire.
En outre, il est également possible d'utiliser à titre de monomères vinyliques (B) les monomères vinyliques polyfonctionnels qui sont exemplifiés par exemple par le triacrylate de triméthylolpropane, le triméthacrylate de triméthylolpropane, le triacrylate de pentaérythritol, le triméthacrylate de pentaérythritol, le diacrylate d'éthylèneglycol, le diméthacrylate d'éthylèneglycol, le diacrylate de tétraéthylèneglycol, le diméthacrylate de tétraéthylèneglycol, le diacrylate de polyéthylèneglycol, le diméthacrylate de polyéthylèneglycol, le diacrylate de 1,4-butanediol, le diméthacrylate de 1,4-butanediol, le diacrylate de 1,6-hexanédiol, le diméthacrylate de 1,6-hexanédiol, le diacrylate de néopentylglycol, le diméthacrylate de néopentylglycol, l'acrylate de triméthylolpropanetrioxyéthyle, le méthacrylate de triméthylolpropanetrioxyéthyle, le diacrylate de tris(2-hydroxyéthyl)isocyanurate, le diméthacrylate de tris(2-hydroxyéthyl)isocyanurate, le triacrylate de tris(2-hydroxyéthyl)isocyanurate, le triméthacrylate de tris(2-hydroxyéthyl)isocyanurate, le polydiméthylsiloxane dont les deux extrémités de la chaîne moléculaire sont bloquées par des groupements alcénylaryle, et d'autres composés siliconés à groupements insaturés.
En ce qui concerne le rapport mentionné ci-dessus dans lequel le composant (A) et le composant (B) sont copolymérisés, le rapport en poids du composé (A) au composé (B) doit être dans la gamme de 0,1:99,9 à 100:0, et, de préférence dans la gamme 1:99 à 100:0.
Le dendrimère carbosiloxane (C) est représenté par la formule générale (III) indiquée ci-dessus. Dans la formule (III), Y est un groupement organique polymérisable par voie radicalaire, dont le type n'est pas sujet à des limitations spéciales quelconques dès lors qu'il s'agit d'un groupement organique susceptible de subir une réaction d'addition radicalaire. Des groupements organiques à acryle et à méthacryle, des groupements organiques à alcénylaryle, ou des groupements alcényle de 2 à 10 atomes de carbone représentés par les formules générales présentées ci-dessous sont suggérées à titre d'exemples spécifiques :

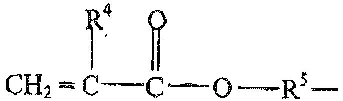

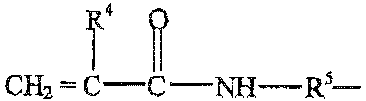

Dans les formules, R⁴ et R⁶ sont des atomes d'hydrogène ou des groupements méthyle, R⁵ et R⁸ sont des groupements alkylène de 1 à 10 atomes de carbone, et R⁷ est un groupement alkyle de 1 à 10 atomes de carbone. L'indice « b » est un entier de 0 à 4, et « c » est 0 ou 1. Acryloxyméthyle, 3-acryloxypropyle, méthacryloxyméthyle, 3-méthacryloxypropyle, 4-vinylphényle, 3-vinylphényle, 4-(2-propényl)phényle, 3-(2-propényl)phényle, 2-)4-vinylphényl)éthyle, 2-(3-vinylphényl)ényle, vinyle, allyle, méthallyle, et 5-hexényle sont suggérés à titre d'exemples de tels groupements organiques polymérisables à voie radicalaire. L'exposant « i » dans la formule (II) qui est un entier de 1 à 10 est le nombre de générations dudit groupement silylalkyle, autrement dit le nombre de fois que le groupement silylalkyle est répété. Ainsi, le dendrimère carboxyloxane de ce composant avec un nombre de génération de 1 est représenté par la formule générale : 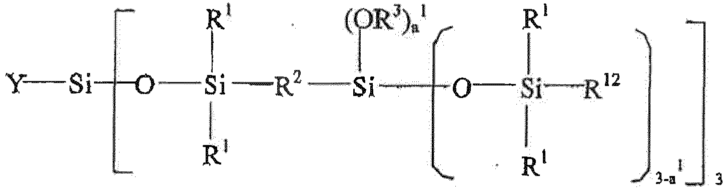 (dans laquelle Y, R¹, R², et R³ sont tels que ci-dessus et R¹² est un atome d'hydrogène ou tel que R¹ décrit ci-dessus. L'indice « a¹ » est un entier de 0 à 3, le total moyen de « a¹ » par molécule étant de 0 à 7). Les dendrimères carbosiloxane de ce composant avec un nombre de génération de 2 sont représentés par la formule générale : 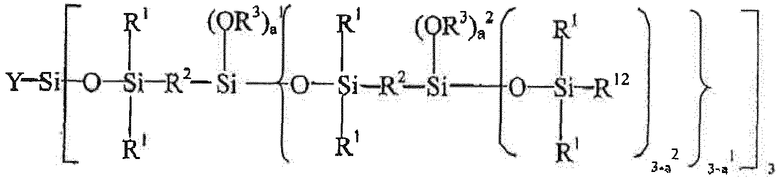 (dans laquelle Y, R¹, R², R³ et R¹² sont tels que ci-dessus et les indices « a¹ » et « a²» sont des entiers de 0 à 3, le total moyen de « a¹» et de « a² » par molécule étant de 0 à 25).
Les dendrimères carbosiloxane de ce composant avec un nombre de génération de 3 sont représentés par la formule générale : 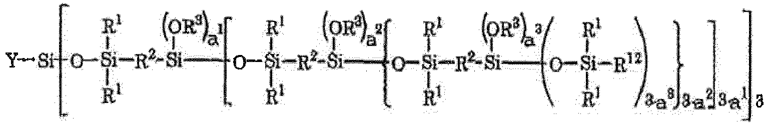 (dans laquelle Y, R¹, R², R³ et R¹² sont tels que ci-dessus et les indices « a¹», « a²» et « a³ » sont des entiers de 0 à 3, le total moyen de « a¹», de « a²» et de « a³» par molécule étant de 0 à 79).

Le composant (C) est exemplifié par des dendrimères carbosiloxane représentés par des formules de composition moyenne représentées ci-dessous :

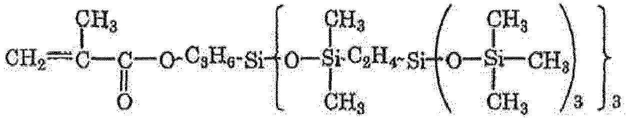

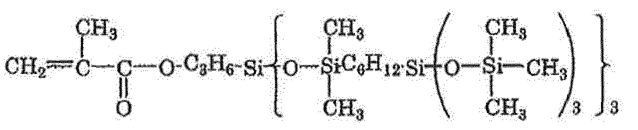

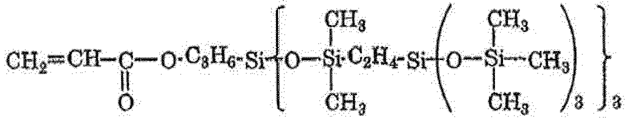

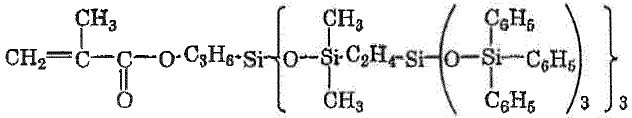

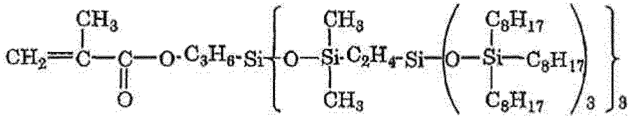

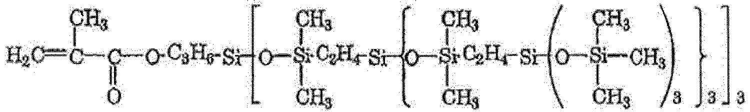

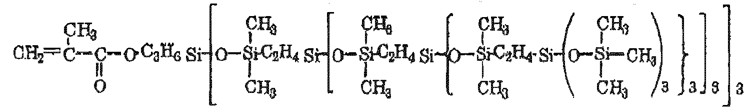

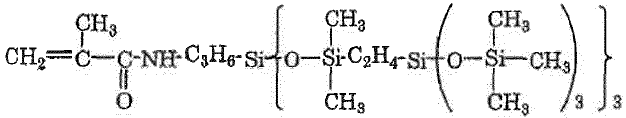

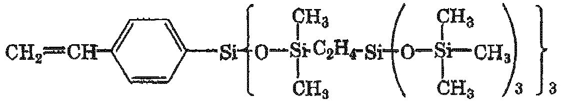

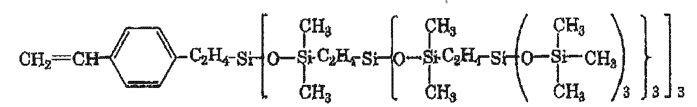

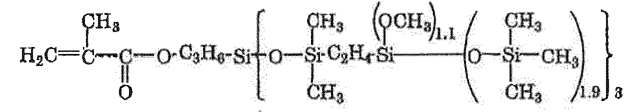

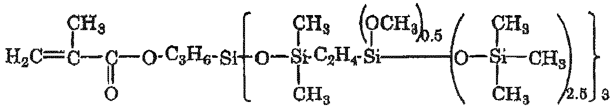

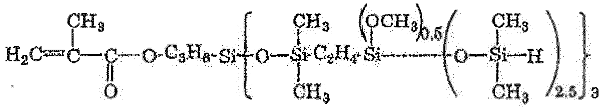

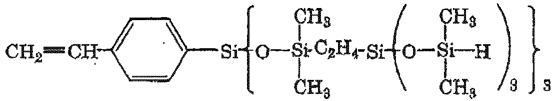

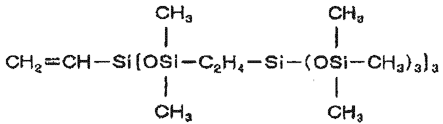

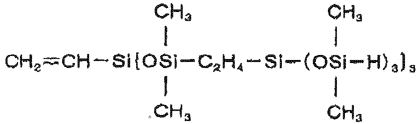

Les dendrimères carbosiloxane du composant (C) peuvent être préparés en utilisant le procédé de préparation pour les copolymères de siloxane/silakylène ramifiés décrits dans le document EP1055674. Par exemple, ils peuvent être préparés en soumettant des composés siliconés organiques à alcényle et des composés siliconés comprenant des atomes d'hydrogène liés au silicium, représentés par la formule générale : 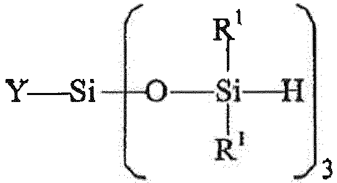 (dans laquelle R¹ et Y, sont tels que ci-dessus) à une réaction d'hydrosilation. Par exemple, le 3-méthacyloxypropyltris(diméthylsiloxy)silane, le 3-acryloxypropyltris(diméthylsiloxy)silane, et le 4-vinylphényltris(diméthylsiloxy)silane sont utilisés à titre de composés de silicium représentés par la formule ci-dessus. Le vinyltris(triméthylsiloxy)silane, le vinyltris(diméthylphénylsiloxy)silane, et le 5-hexényltris(triméthylsiloxy)silane sont utilisés comme composés organiques de silicium à alcényle. En outre, il est préférable d'effectuer la réaction d'hydrosilation en présence d'un catalyseur de métal de transition tel que l'acide chloroplatinique et le complexe de platine/vinylsiloxane.
Le rapport de copolymérisation du composant (C), en termes de son rapport en poids par rapport au total du composé (A) et du composé (B), doit être dans la gamme de 0,1:99,9 à 99,9 :0,1, et de préférence dans la gamme de 1:99 à 99:1, et encore plus préférablement dans la gamme de 5:95 à 95:5.
Des groupements amino peuvent être introduits dans les chaînes latérales du polymère vinylique en utilisant, inclus dans le composant (B), des monomères vinyliques contenant des groupements amino, tels que l'acrylate de diméthylaminoéthyle, le méthacrylate de diméthylaminoéthyle, l'acrylate de diéthylaminoéthyle et le méthacrylate de diéthylaminoéthyle, puis en réalisant une modification avec du monochlorure acétate de potassium, du monochlorure acétate d'ammonium, du sel d'aminométhylpropanol d'acide monochloroacétique, du sel de triéthanolamine d'acide monobromoacétique, du monochloropropionate de sodium, et d'autres sels de métaux alcalins d'acides gras halogénés ; sinon on peut introduire des groupements acides carboxyliques dans les chaînes latérales du polymère vinylique en utilisant, inclus dans le composant (B), des monomères vinyliques contenant des acides carboxyliques, tels que l'acide acrylique, l'acide méthacrylique, l'acide itaconique, l'acide crotonique, l'acide fumarique et l'acide maléique, et similaires, puis en neutralisant le produit avec de la triéthylamine, de la diéthylamine, de la triéthanolamine, et d'autres aminés.

Le polymère vinylique fluoré peut être un des polymères décrits dans les exemples de la demande WO03/045337.

Selon un mode préféré de réalisation, les polymères vinyliques greffés au sens de la présente invention sont véhiculés dans une huile, de préférence volatile, choisie parmi les huiles de silicones, et/ou les huiles hydrocarbonées.
Selon un mode particulier de réalisation, l'huile de silicone peut être la cyclopentasiloxane.
Selon un autre mode particulier de réalisation, l'huile hydrocarbonée peut être l'isododécane.
Les polymères vinyliques greffés avec au moins un motif dérivé de dendrimère carbosiloxane pouvant convenir particulièrement à la présente invention sont les polymères vendus sous les dénominations TIB 4-100, TIB 4-101, TIB 4-120, TIB 4-130, TIB 4-200, FA 4002 ID (TIB 4-202), TIB 4-220, FA 4001 CM (TIB 4-230) par la société Dow Corning.

Le polymère vinylique ayant au moins un motif dérivé de dendrimère carbosiloxane peut être présent en une teneur en matière active allant de 0,1 % à 30 % en poids, par rapport au poids total de la composition, de préférence allant de 0,5 % à 20 % en poids, et préférentiellement allant de 1 % à 10 % en poids, de préférence encore allant de 2 à 7% en poids.

### Copolymère d'oléfine

La composition selon l'invention comprend au moins un copolymère d'oléfine choisi parmi les copolymères d'oléfine amorphes ou les copolymères d'oléfine à cristallisation contrôlée et modérée.

Par copolymère d'oléfine au sens de la présente demande, on entend tout copolymère formé par polymérisation d'au moins une oléfine et d'un autre monomère additionnel différent de ladite oléfine.

L'oléfine peut être notamment un monomère à insaturation éthylénique.
Comme exemple d'oléfine, on peut citer les monomères de carbure éthylénique, ayant notamment une ou deux insaturation éthylénique, ayant de 2 à 5 atomes de carbone tels que l'éthylène, le propylène, le butadiène, l'isoprène.

Selon un aspect particulier de l'invention, le copolymère bloc est un copolymère bloc hydrocarboné formé par polymérisation de monomères de carbure éthylénique, ayant notamment une ou deux insaturation éthyléniques, ayant de 2 à 5 atomes de carbone.

Selon un aspect de l'invention, le copolymère bloc hydrocarboné est formé par polymérisation d'oléfine choisie parmi l'éthylène, le propylène, le butadiène, l'isoprène.

### Copolymère d'oléfine amorphe

Selon un premier mode de réalisation, le copolymère d'oléfine peut être un copolymère amorphe formé par polymérisation d'au moins une oléfine.

Par copolymère amorphe, on entend un polymère qui n'a pas de forme cristalline. Le copolymère amorphe est également filmogène, c'est-à-dire qu'il est capable de former un film lors de son application sur la peau.

Le copolymère d'oléfine amorphe peut être notamment un copolymère dibloc, tribloc, multibloc, radial, étoile, ou leurs mélanges.
De tels copolymères d'oléfine amorphes sont décrits dans la demande US-A-2002/005562 et dans le brevet US-A-5 221 534.
Avantageusement, le copolymère d'oléfine amorphe est un copolymère bloc amorphe de styrène et d'oléfine.

Le copolymère d'oléfine amorphe est de préférence hydrogéné pour réduire les insaturations éthyléniques résiduelles après la polymérisation des monomères.

En particulier, le copolymère d'oléfine amorphe est un copolymère, éventuellement hydrogéné, à blocs styrène et à blocs éthylène/alkylène en C3-C4.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme copolymère d'oléfine amorphe un copolymère dibloc tel que ceux décrits précédemment, en particulier un copolymère dibloc de styrène-éthylène/propylène.

### Copolymère d'oléfines à cristallisation contrôlée et modérée

Selon un second mode de réalisation, le copolymère d'oléfine est un copolymère d'oléfines à cristallisation contrôlée et modérée.

Les copolymères d'oléfines à cristallisation contrôlée et modérée utilisés dans la composition de la présente demande peuvent être tout copolymère d'oléfines, à savoir un copolymère comportant uniquement des motifs oléfiniques, ayant un caractère cristallin contrôlé et modéré, c'est-à-dire un taux de cristallinité au plus égal à 50%, de préférence allant de 5 à 40%, et mieux allant de 10 à 35%.
Ces copolymères sont généralement des élastomères ou des plastomères et peuvent être synthétisés par tout procédé connu, en particulier par voie radicalaire, par catalyse Ziegler-Natta ou par catalyse métallocène. De tels polymères sont notamment décrits dans la demande EP-A-1 034 776.

Une première classe de copolymères d'oléfines à cristallisation contrôlée et modérée, utilisables dans les compositions selon l'invention, peuvent être les copolymères d'α-oléfine linéaire ou ramifiée, en particulier d'α-oléfine en C₂-C₁₆ et mieux en C₂-C₁₂. De préférence, ces copolymères sont des bi- ou terpolymères et tout particulièrement des bipolymères.
Parmi les bipolymères recommandés pour les compositions de l'invention, on peut citer les bipolymères d'éthylène et d'α-oléfine en C₄-C₁₆, de préférence en C₄-C₁₂ et les bipolymères de propylène et d'α-oléfine en C₄-C₁₆, de préférence en C₄-C₁₂. De préférence encore, l'α-oléfine est choisie parmi le butène-1, le pentène-1, l'hexène-1, l'octène-1, le nonène-1, le décène-1, l'undécène-1, le dodécène-1, le 3,5,5-triméthylhexène-1, le 3-méthylpentène-1, et le 4-méthylpentène-1.
Parmi ces monomères, le butène-1 et l'octène-1 sont particulièrement préférés. Les bipolymères recommandés sont les élastomères ayant un taux de cristallinité allant de 10 à 35 %.
Ces bipolymères sont préférentiellement synthétisés par catalyse métallocène. De tels bipolymères sont commercialisés par la Société DOW CHEMICAL sous les dénominations commerciales « AFFINITY » (plastomères) et par la Société Dupont de Nemours sous la dénomination « ENGAGE » (élastomères).
Des bipolymères éthylène-butène sont commercialisés par la Société EXXON sous l'appellation commerciale « EXACT RESINS » et par la société ELENAC sous l'appellation commerciale « LUFLEXEN ».

Parmi les terpolymères, on peut citer les terpolymères d'éthylène, de propylène et d'α-oléfine en C₄-C₁₆, de préférence C₄-C₁₂.
Dans ces terpolymères, les teneurs en α-oléfine en C₄-C₁₆ sont comme indiquées précédemment et les α-oléfines préférées sont le butène, l'hexène et l'octène.
Les copolymères préférés, décrits dans la demande EP-A-1 034 776, peuvent en particulier être des copolymères d'éthylène/octène vendu sous la référence « Engage 8400 » par la société Dupont de Nemours.

Une seconde classe de copolymères d'oléfines à cristallisation contrôlée et modérée convenant pour l'invention sont les copolymères d'éthylène ou de propylène et d'une cyclooléfine, en particulier les bipolymères.

Généralement, la teneur en cyclooléfine des copolymères est inférieure à 20% en mole.

Parmi les cyclooléfines utilisables, on peut citer le cyclobutène, le cyclohexène, le cyclooctadiène, le norbornène, le diméthano-octahydronaphtalène (DMON), l'éthylidène norbornène, le vinyl norbornène et le 4-vinylcyclohexène.

Les copolymères recommandés de cette classe sont les copolymères d'éthylène et de norbornène. La teneur en norbornène de ces copolymères est généralement inférieure à 18% en mole pour présenter le caractère cristallin requis et ces copolymères sont synthétisés par catalyse métallocène.

Des copolymères éthylène/norbornène appropriés sont commercialisés par les Sociétés MITSUI PETROCHEMICAL ou MITSUI-SEKKA sous la dénomination commerciale « APPEL » et par la Société HOECHST-CELANESE sous la dénomination commerciale « TOPAS ».

D'autres copolymères d'éthylène/cyclooléfine recommandés sont les bipolymères éthylène/cyclobutène et éthylène/cyclohexène à faible teneur en cyclooléfine, généralement inférieure à 20% en mole.

Une troisième classe de copolymères d'oléfines convenant pour la présente invention, est constituée par les copolymères de monooléfine et de monomère à liaison(s) éthylénique(s) tel que les diènes, par exemple les bipolymères éthylène/butadiène, propylène/butadiène, éthylène/isoprène et propylène/isoprène, et les terpolymères éthylène/propylène/diène, obtenus également par synthèse métallocène.

La proportion de motifs « éthylène » ou « diène » dans le copolymère à cristallisation contrôlée est généralement comprise dans la gamme allant de 3 à 20% en mole.

Selon un mode préféré de réalisation, le copolymère d'oléfine à cristallisation contrôlée et modérée est choisi parmi les copolymères d'éthylène/octène et les copolymères éthylène/norbornène.

Selon un mode préféré de réalisation, le copolymère d'oléfine peut notamment être un gélifiant polymérique est capable d'épaissir ou de gélifier la phase organique de la composition.

Selon un mode préféré de réalisation, le copolymère d'oléfine peut notamment être filmogène. Par polymère filmogène au sens de la présente demande, on entend un polymère apte à former à lui seul ou à l'aide d'un agent auxiliaire de filmification, un film macroscopiquement continu sur un support, notamment sur les matières kératiniques, de préférence un film cohésif et mieux encore un film dont la cohésion et les propriétés mécaniques sont telles que ledit film peut être isolé dudit support.

Selon un mode préféré de réalisation, le copolymère d'oléfine est choisi parmi les copolymères d'oléfine amorphes, notamment parmi les copolymères diblocs tel que ceux de styrène-éthylène/propylène décrits précédemment.

Le copolymère d'oléfine peut être présent dans la composition selon l'invention en une teneur allant de 0,1 % à 10 % en poids, par rapport au poids total de la composition, de préférence allant de 0,2 % à 5 % en poids, et préférentiellement allant de 0,5 % à 3 % en poids.

Selon un mode préféré de réalisation, la composition selon l'invention comprend :
- un polymère vinylique greffé avec un dendrimère carbosiloxane qui est le produit de polymérisation:
   (A) de 0,1 à 99 partie en poids d'un ou plusieurs monomère(s) acrylate ou méthacrylate; et
   (B) de 100 à 0,1 parties en poids d'un monomère acrylate ou méthacrylate d'un dendrimère carbosiloxane tri[tri(triméthylsiloxy)silyléthyl diméthylsiloxy]silylpropyle, le dendrimère carbosiloxane tri[tri(triméthylsiloxy)silyléthyl diméthylsiloxy]silylpropyle répondant à l'une des formules : 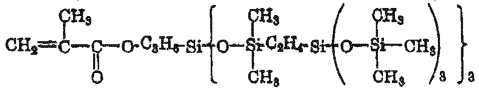 ou 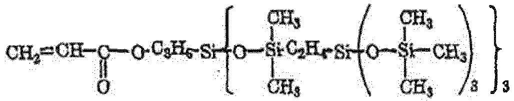 et,
- un copolymère d'oléfine choisi parmi les copolymères d'oléfine amorphes, notamment parmi les copolymères diblocs tel que ceux de styrène-éthylène/propylène décrits précédemment.

### Phase grasse

La composition selon l'invention comprend une phase grasse liquide.

La phase grasse liquide comprend au moins une huile choisie parmi les huiles volatiles, les huiles non volatiles, et leurs mélanges.

Selon un mode préféré de réalisation, la composition selon l'invention peut comprendre au moins une huile volatile.

Par « huile volatile », on entend au sens de l'invention toute huile susceptible de s'évaporer au contact de la peau, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,13 Pa à 40.000 Pa (0,001 à 300 mm de Hg) et de préférence allant de 1,3 à 1300 Pa (0,01 à 10 mm de Hg).

L'huile volatile peut être choisie parmi les huiles volatiles hydrocarbonées, les huiles volatiles siliconées, les huiles volatiles fluorées, et leurs mélanges.

Selon un mode préféré de réalisation, la composition selon l'invention peut comprendre au moins une huile volatile hydrocarbonée.

On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars^{®} ou de Permethyls^{®}.

Comme huiles volatiles, on peut aussi utiliser les silicones volatiles, comme par exemple les huiles de silicones linéaires ou cycliques volatiles, notamment celles ayant une viscosité ≤ 5 centistokes (5 x 10⁻⁶ m²/s), et ayant notamment de 2 à 10 atomes de silicium, de préférence de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile fluorée n'a généralement pas de point éclair.
Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Selon un mode préféré de réalisation, la composition selon l'invention peut comprendre au moins deux huiles volatiles différentes, de préférence au moins trois huiles volatiles différentes.

En particulier, la composition selon l'invention peut comprendre de l'isododécane, de la cyclopentasiloxane ; de l'isohéxadécane ou leur mélange.

La composition selon l'invention peut comprendre une huile volatile en une teneur allant de 1 à 80 % en poids par rapport au poids total de la composition, de préférence de 5 à 70 % en poids, plus préférentiellement allant de 10 à 60 % en poids, et plus préférentiellement encore de 15 à 50% en poids.

La composition selon l'invention peut comprendre au moins une huile non volatile.

Par "huile non volatile", on entend une huile restant sur la peau à température ambiante et pression atmosphérique au moins plusieurs heures et ayant notamment une pression de vapeur inférieure à 0,13 Pa (0,01 mm de Hg).

Ces huiles non volatiles peuvent être des huiles hydrocarbonées notamment d'origine animale ou végétale, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment des triglycérides d'acide heptanoïque ou d'acide octanoïque, ou bien encore les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810^{®}, 812^{®} et 818^{®} par la société Dynamit Nobel,

- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le Parleam ®, le squalane, les huiles de paraffine, et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le néopentanoate d'isodecyl, le palmitate de 2-éthyl-hexyle, l'isostéarate d'isostéaryle, le laurate de 2-hexyl-décyle, le palmitate de 2-octyl-décyle, le myristate de 2-octyl-dodécyle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle, le lactate de 2-octyl-dodécyle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et leurs mélanges.

L'huile non volatile peut être présente dans la composition selon l'invention en une teneur allant de 0,5 % à 80 % en poids, par rapport au poids total de la composition, de préférence allant de 1 % à 50 % en poids, de préférence allant de 2 % à 30 % en poids.

La phase grasse liquide peut être présente dans la composition selon l'invention en une teneur totale allant de 1 à 90 % en poids par rapport au poids total de la composition, de préférence de 5 à 80 % en poids, plus préférentiellement allant de 10 à 70 % en poids, et plus préférentiellement encore de 25 à 60 % en poids.

La phase grasse de la composition selon l'invention peut également comprendre des corps gras autres que les huiles citées ci-dessus, comme des cires ou encore des corps gras pâteux.

Par cires on entend un corps gras solide à température ambiante.

On peut définir les corps gras pâteux à l'aide d'au moins une des propriétés physico-chimiques suivantes :
- une viscosité de 0,1 à 40 Pa.s (1 à 400 poises), mesurée à 40 °C avec un viscosimètre rotatif CONTRAVES TV équipé d'un mobile MS-r3 ou MS-r4 à la fréquence de 60 Hz,
- un point de fusion de 25-70 °C, de préférence 25-55 °C.

A titre de cires pouvant être utilisées selon l'invention, on peut citer :
- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la C₃₀-C₄₅ alkyl méthicone vendue sous la dénomination commerciale "AMS C 30" par DOW CORNING,
- les huiles hydrogénées concrètes à 25°C telles que l'huile de ricin hydrogénée, l'huile de jojoba hydrogénée, l'huile de palme hydrogénée, le suif hydrogéné, l'huile de coco hydrogénée et les esters gras concrets à 25°C comme le stéarate d'alkyle en C₂₀-C₄₀ vendu sous la dénomination commerciale "KESTER WAX K82H" par la société KOSTER KEUNEN,
- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.

De préférence, les cires sont présentes à une teneur allant de 0,1 à 30% en poids, de préférence encore de 0,5 à 20 % en poids, par rapport au poids total de la composition.

Ces corps gras peuvent en particulier être choisis de manière variée par l'homme du métier afin de préparer une composition ayant les propriétés souhaitées, par exemple en consistance ou en texture.

### Agent épaississant

La composition selon l'invention peut comprendre, outre le copolymère d'oléfine, au moins un agent épaississant des huiles choisi parmi les agents épaississants polymériques, les agents épaississants minéraux et leurs mélanges.

L'agent épaississant polymérique des huiles est capable d'épaissir ou de gélifier la phase organique de la composition. L'agent épaississant polymérique peut être également filmogène, c'est-à-dire qu'il est capable de former un film lors de son application sur la peau.

L'agent épaississant polymérique des huiles peut notamment être choisi parmi:
- les polycondensats de type polyamide résultant de la condensation entre (α) au moins un acide choisi parmi les acides dicarboxyliques comprenant au moins 32 atomes de carbone tels que les acides gras dimères et (β) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG^{®} par la société ARIZONA CHEMICAL ;
- les polymères siliconés du type :
   1) des polyorganosiloxanes comportant au moins deux groupes capables d'établir des interactions hydrogène, ces deux groupes étant situés dans la chaîne du polymère, et/ou
   2) des polyorganosiloxanes comportant au moins deux groupes capables d'établir des interactions hydrogène, ces deux groupes étant situés sur des greffons ou ramifications.
Les groupes capables d'établir des interactions hydrogène peuvent être choisis parmi les groupes ester, amide, sulfonamide, carbamate, thiocarbamate, urée, uréthane, thiourée, oxamido, guanidino, biguanidino et leurs combinaisons.

Les polymères siliconés utilisés comme agents structurants dans la composition de l'invention sont des polymères du type polyorganosiloxane comme par exemple ceux décrits dans les documents US-A-5 874 069, US-A-5,919,441, US-A-6,051,216 et US-A-5,981,680.
En particulier, les polymères siliconés sont des polyorganosiloxanes tels que définis ci-dessus et dont les motifs capables d'établir des interactions hydrogènes sont disposés dans la chaîne du polymère.
Les polymères siliconés peuvent plus particulièrement être des polymères comprenant au moins un motif répondant à la formule générale I : 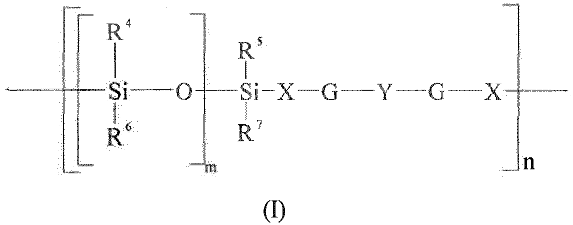 dans laquelle :
1) R⁴, R⁵, R⁶ et R⁷, identiques ou différents, représentent un groupe choisi parmi:
   - les groupes hydrocarbonés, linéaires, ramifiés ou cycliques, en C₁ à C₄₀, saturés ou insaturés, pouvant contenir dans leur chaîne un ou plusieurs atomes d'oxygène, de soufre et/ou d'azote, et pouvant être substitués en partie ou totalement par des atomes de fluor,
   - les groupes aryles en C₆ à C₁₀, éventuellement substitués par un ou plusieurs groupes alkyle en C₁ à C₄,
   - les chaînes polyorganosiloxanes contenant ou non un ou plusieurs atomes d'oxygène, de soufre et/ou d'azote,
2) les X, identiques ou différents, représentent un groupe alkylène di-yle, linéaire ou ramifié en C₁ à C₃₀, pouvant contenir dans sa chaîne un ou plusieurs atomes d'oxygène et/ou d'azote,
3) Y est un groupe divalent alkylène linéaire ou ramifié, arylène, cycloalkylène, alkylarylène ou arylalkylène, saturé ou insaturé, en C₁ à C₅₀, pouvant comporter un ou plusieurs atomes d'oxygène, de soufre et/ou d'azote, et/ou porter comme substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyle en C₁ à C₃, hydroxyalkyle en C₁ à C₃ et amino alkyle en C₁ à C₆, ou
4) Y représente un groupe répondant à la formule : 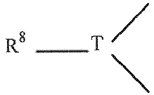 dans laquelle
   - T représente un groupe hydrocarboné trivalent ou tétravalent, linéaire ou ramifié, saturé ou insaturé, en C₃ à C₂₄ éventuellement substitué par une chaîne polyorganosiloxane, et pouvant contenir un ou plusieurs atomes choisis parmi O, N et S, ou T représente un atome trivalent choisi parmi N, P et Al, et
   - R³ représente un groupe alkyle en C₁ à C₅₀, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,
5) les G, identiques ou différents, représentent les groupes divalents choisis parmi: 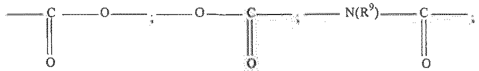 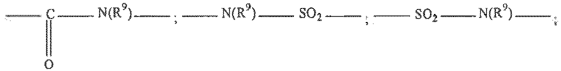 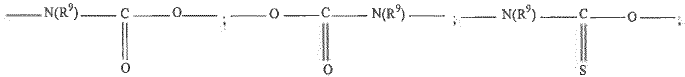 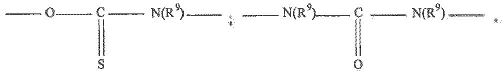 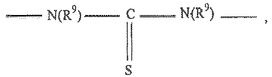 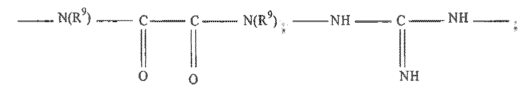 et 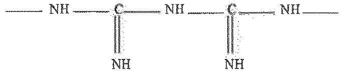 où R⁹ représente un atome d'hydrogène ou un groupe alkyle, linéaire ou ramifié, en C₁ à C₂₀, à condition qu'au moins 50 % des R⁹ du polymère représente un atome d'hydrogène et qu'au moins deux des groupes G du polymère soient un autre groupe que : 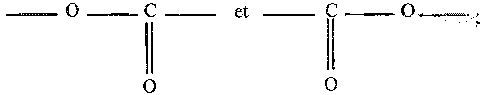
6) n est un nombre entier allant de 2 à 500, de préférence de 2 à 200, et m est un nombre entier allant de 1 à 1000, de préférence de 1 à 700 et mieux encore de 6 à 200.

Selon l'invention, 80 % des R⁴, R⁵, R⁶ et R⁷, du polymère sont choisis de préférence parmi les groupes méthyle, éthyle, phényle et 3,3,3-trifluoropropyle.
Selon un mode de réalisation avantageux, les groupes capables d'établir des interactions hydrogènes sont des groupes amides de formule -C(O)NH- et -HN-C(O)-. Dans ce cas, l'agent structurant peut être un polymère comprenant au moins un motif de formule (III) ou (IV) : 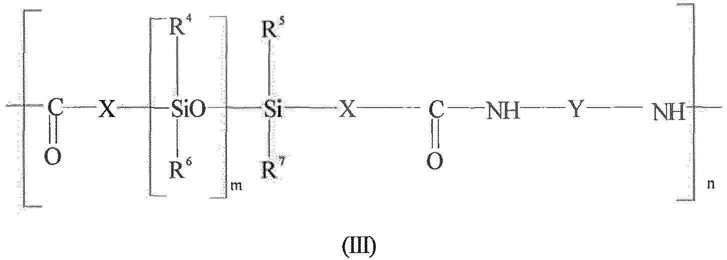 ou 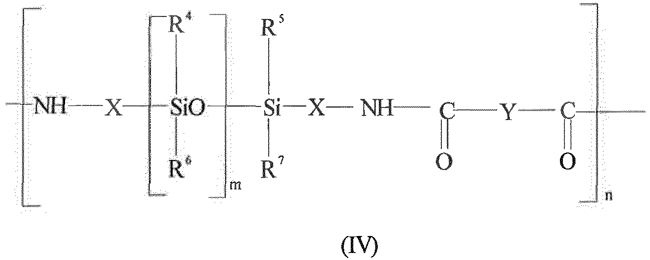 dans lesquelles R⁴, R⁵, R⁶, R⁷, X, Y, m et n sont tels que définis ci-dessus. Dans ces polyamides de formule (III) ou (IV), m va de 1 à 700, en particulier de 15 à 500 et notamment de 50 à 200 et n va particulier de 1 à 500, de préférence de 1 à 100 et mieux encore de 4 à 25,
- X est de préférence une chaîne alkylène linéaire ou ramifiée ayant de 1 à 30 atomes de carbone, en particulier 1 à 20 atomes de carbone, notamment de 5 à 15 atomes de carbone et plus particulièrement de 10 atomes de carbone, et
- Y est de préférence une chaîne alkylène linéaire ou ramifiée ou pouvant comporter des cycles et/ou des insaturations, ayant de 1 à 40 atomes de carbone, en particulier de 1 à 20 atomes de carbone, et mieux encore de 2 à 6 atomes de carbone, en particulier de 6 atomes de carbone.
- les galactomananes comportant de un à six, et en particulier de deux à quatre, groupes hydroxyle par ose, substitués par une chaîne alkyle saturée ou non, comme la gomme de guar alkylée par des chaînes alkyle en C₁ à C₆, et en particulier en C₁ à C₃ et leurs mélanges ;

La composition selon l'invention peut également comprendre au moins un agent épaississant minéral des huiles tel qu'une argile organophile, les silices pyrogénées.

Les argiles organophiles sont des argiles modifiées par des composés chimiques rendant l'argile apte à gonfler dans les milieux huileux.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.
Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques comme la peau.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Bentone ISD V par la société Elementis, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6^{ème} édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6^{ème} édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

Selon un mode préféré de réalisation, la composition selon l'invention peut comprendre l'association d'une silice pyrogénée hydrophobe et de quaternium-18/benzalkonium bentonite

L'agent épaississant des huiles peut être présent dans la composition selon l'invention en une teneur en matière active allant de 0,01 % à 15 % en poids, par rapport au poids total de la composition, de préférence en une teneur allant de 0,1 % à 10 % en poids, et préférentiellement allant de 0,3 % à 5 % en poids.

### Phase aqueuse

La composition selon l'invention peut comprendre une phase aqueuse.

La phase aqueuse comprend de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

La phase aqueuse peut également comprendre des solvants organiques miscibles à l'eau (à température ambiante - 25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ; les éthers de glycol (ayant notamment de 3 à 16 atomes de carbone) tels que les alkyl(C1-C4)éther de mono, di- ou tripropylène glycol, les alkyl(C1-C4)éthers de mono, di- ou triéthylène glycol, le carylyl glycol et leurs mélanges.

La phase aqueuse peut comprendre en outre des agents de stabilisation, par exemple le chlorure de sodium, le dichlorure de magnésium et le sulfate de magnésium.

La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.

De préférence, la phase aqueuse peut être présente dans la composition selon l'invention en une teneur allant de 1 à 80 % en poids, par rapport au poids total de la composition, et de préférence de 5 à 50 % en poids, et plus préférentiellement de 5 à 40 % en poids.

### Phase pulvérulente

La composition selon l'invention peut comprendre une phase pulvérulente notamment choisie parmi les pigments, les charges et/ou les nacres et leurs mélanges.

Selon un mode préféré de réalisation, la composition selon l'invention peut comprendre des pigments.

Par « pigments », il faut comprendre des particules, minérales ou organiques, insolubles dans la phase organique liquide, destinées à colorer et/ou opacifier la composition.

Les pigments peuvent être des pigments minéraux ou organiques. Comme pigments, on peut utiliser les oxydes métalliques comme les oxydes de fer (notamment ceux de couleur jaune, rouge, brun, noir), les dioxydes de titane, l'oxyde de cérium, l'oxyde de zirconium, l'oxyde de chrome ; le violet de manganèse, l'ultramarine bleue, le bleu de prusse, le bleu outremer, le bleu ferrique et leurs mélanges.
On utilise de préférence des pigments d'oxydes de fer et/ou de dioxyde de titane.

Les pigments peuvent être traités avec un agent hydrophobe pour les rendre compatible avec la phase organique de la composition. L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.
Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zin, de sodium, de potassium. L'acide aminé peut être par exemple la lysine, l'acide glutamique, l'alanine.

Le terme alkyl mentionné dans les composés cités précédemment désigne notamment un groupe alkyle ayant de 1 à 30 atomes de carbone, de préférence ayant de 5 à 16 atomes de carbone.
Des pigments traités hydrophobes sont notamment décrits dans la demande EP-A-1086683.

Les pigments peuvent être présents, dans la composition selon l'invention, en une teneur allant de 0,1 à 40% en poids, par rapport au poids total de la composition, en particulier allant de 1% à 30% en poids, et plus préférentiellement allant de 5 % à 15% en poids.

Outre les pigments, la phase pulvérulente de la composition selon l'invention peut comprendre des charges et/ou des nacres.

Selon un mode préféré de réalisation, la composition selon l'invention peut comprendre des charges.

Par charges, il faut comprendre des particules de toute forme, incolores ou blanches, minérales ou de synthèse, insolubles dans le milieu de la composition quelle que soit la température à laquelle la composition est fabriquée.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®), les poudres de poly-β-alanine, les poudres de polyéthylène, les polyméthacrylates de méthyle, les poudres de polyuréthane telle que la poudre de copolymère de diisocyanate d'hexaméthylène et de triméthylol hexyl lactone vendue sous les dénominations PLASTIC POWDER D-400 par la société TOSHIKI, les poudres de polymères de tétrafluoroéthylène (Téflon®), les particules de cire micronisée notamment les microcires de Carnauba telles que celles commercialisées sous la dénomination de « MicroCare 350^{®} » par la société MICRO POWDERS, les microcires de cire synthétique telles que celles commercialisées sous la dénomination de « MicroEase 114S^{®} » par la société MICRO POWDERS, les microcires constituées d'un mélange de cire de Carnauba et de cire de polyéthylène telles que celles commercialisées sous les dénominations de « MicroCare 300^{®} » et « 310^{®} » par la société MICRO POWDERS, les microcires constituées d'un mélange de cire de Carnauba et de cire synthétique telles que celles commercialisées sous la dénomination de « MicroCare 325^{®} » par la société MICRO POWDERS, les microcires de polyéthylène telles que celles commercialisées sous les dénominations de « MicroPoly 200^{®} », « 220^{®} », « 220L^{®} », et « 250S^{®} », par la société MICRO POWDERS, et celles commercialisées sous la dénomination de « CERAPURE H5-C » par la société SHAMROCK, ou les microcires de polypropylène telle que celles commercialisées sous la dénomination « MATTEWAX » par la société MICRO POWDERS ; la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique, les poudres de résine de silicone, en particulier les poudres de silsesquioxane (poudres de résine de silicone notamment décrites dans le brevet EP 293795 ; Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses, les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; le sulfate de baryum et leurs mélanges.

Selon un mode préféré de réalisation, la composition selon l'invention peut comprendre une poudre de polytétrafluoroéthylène (PTFE), une poudre de polyamide (Nylon®) ou leur mélange.

Les charges peuvent être présentes dans la composition selon l'invention en une teneur totale allant de 0,1 % à 30 % en poids, par rapport au poids total de la composition, de préférence allant de 0,5 % à 20 % en poids, et préférentiellement allant de 0,8 % à 10 % en poids.

Outre les pigments et les charges, la phase particulaire de la composition selon l'invention peut comprendre des nacres.

Par « nacres », il faut comprendre des particules irisées, notamment produites par certains mollusques dans leur coquille ou bien synthétisées, insolubles dans le milieu de la composition.

Les nacres peuvent être choisies parmi les pigments nacrés blancs tels que l'oxychlorure de bismuth, le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

### Colorants additionnels

La composition selon l'invention peut comprendre des colorants additionnels choisis parmi les colorants hydrosolubles et liposolubles.

Les colorants hydrosolubles sont par exemple le jus de betterave, le bleu de méthylène, le caramel.

Par colorants liposolubles, il faut comprendre des composés généralement organiques solubles dans les corps gras comme les huiles.

Les colorants liposolubles sont par exemple le rouge Soudan, le D&C Red n° 17, le D&C Green n° 6, le β-carotène, l'huile de soja, le brun Soudan, le D&C Yellow n° 11, le D&C Violet n° 2, le D&C orange n° 5, le jaune quinoléine, le rocou, les bromoacides.

### Galéniques

La composition selon l'invention peut se présenter sous différentes formes galéniques, notamment anhydre ou émulsions eau-dans-huile, huile-dans-eau ou multiples, et se présenter sous forme de gel, crèmes, suspensions, compact, coulés à chaud et sticks. La présente invention peut également se présenter sous a forme d'un kit de maquillage et/ou de soin des matières kératiniques comprenant au moins une première composition selon l'invention comprenant i) au moins un polymère vinylique ayant au moins un motif dérivé de dendrimère carbosiloxane et, ii) au moins un copolymère d'oléfine, et au moins une seconde composition cosmétique.
Le kit peut comprendre deux compositions distinctes destinées à être appliquées l'une sur l'autre, l'une desdites compositions comprenant au moins au moins un polymère vinylique ayant au moins un motif dérivé de dendrimère carbosiloxane et l'autre composition comprenant au moins un copolymère d'oléfine.

Selon un mode préféré de réalisation, la composition selon l'invention peut se présenter sous forme anhydre ou sous forme d'émulsion, préférentiellement eau-dans-huile.

### Ingrédients cosmétiques usuels additionnels

La composition selon l'invention peut comprendre au moins un autre ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les sequestrants, les agents filmogènes, et leurs mélanges.

L'invention est présentée plus en détail dans les exemples ci-après.

### Exemples 1 à 7 :

On a préparé 7 fonds de teint sous forme d'émulsion eau-dans-huile ayant la formule générale suivante :

| | | %massique |
|---|---|---|
| A1 | Polydiméthylesiloxane alpha-omega oxyéthyléné/ oxypropyléné dans cyclopentasiloxane vendu sous la dénomination Abil EM 97 par la Société Goldschmidt | 1,80 |
| | Isostearyl diglyceryl succinate | 0,60 |
| | Butyl paraben | 0,25 |
| | Isododécane | X |
| | | |
| A2 | Isododécane | 15,35 |
| | Copolymère bloc styrène- éthylène/propylène vendu sous la référence KRATON G1701 E par la Société Kraton Polymers | Z |
| | | |
| A3 | Cyclopentasiloxane | 5,00 |
| | Oxydes de fer enrobés de stearoyl glutamate d'aluminium | 3,2 |
| | Dioxyde de titane enrobé de stearoyl glutamate d'aluminium | 6,8 |
| | | |
| A4 | Poudre de polytétrafluoroéthylène (PTFE) | 1,4 |
| | Poudre de nylon 12 | 1,1 |
| | | |
| B | Eau déminéralisée | 25,25 |
| | Octane 1-2 diol | 0,3 |
| | Glycérol | 3,0 |
| | Conservateurs | 0,75 |
| | Sulfate de Magnésium | 0,70 |
| C | Copolymère vinylique contenant des chaînes latérales silicones dendritiques dans l'isododécane vendu sous la dénomination FA 4002 ID par la société Dow Corning. | Y |
| | | |
| D | Silicate de magnésium modifié dans l'isododécane vendu sous la dénomination Bentone Gel ISD V par la société Elementis | 8,00 |
| | TOTAL | 100% |

Les fonds de teint des exemples 1, 2, 5, 6 et 7 sont des fonds de teint selon l'invention et les fonds de teint des exemples 3 et 4 sont des comparatifs dans la mesure où ils sont exempts respectivement de polymère vinylique ayant un motif dérivé de dendrimère carbosiloxane ou de copolymère d'oléfine.

| | Ex 1 (invention) | Ex 2 (invention) | Ex 3 (comparaison) | Ex 4 (comparaison) |
|---|---|---|---|---|
| X (Isododécane A1) | 17,5 | 12,5 | 25,0 | 14,0 |
| Y (polymère vinylique avec motif dendrimère carbosiloxane) | 7,5 | 12,5 | 0 | 12,5 |
| Z (copolymère d'oléfine) | 1,50 | 1,50 | 1,50 | 0 |

| | Ex 5 (invention) | Ex 6 (invention) | Ex 7 (invention) |
|---|---|---|---|
| X (Isododécane A1) | 18,1 | 13,1 | 0,6 |
| Y (polymère vinylique avec motif dendrimère carbosiloxane) | 7,5 | 12,5 | 25,0 |
| Z (copolymère d'oléfine) | 0,90 | 0,90 | 0,90 |

### Mode opératoire :

### 1. Préparation des phases :

Préparation de A1 : on pèse les trois premiers constituants et on chauffe le mélange jusqu'à 75 °C de manière à obtenir un milieu limpide puis on ajoute l'isododécane.
Préparation de A2: dans un poêlon on mélange, sous agitation (Rayneri), le copolymère et l'isododécane que l'on chauffe à 90°C jusqu'à ce que le milieu soit homogène.
Préparation de A3: on broie les pigments à la tricylindre (3 passages) dans la cyclopentasiloxane.
Préparation de B: on pèse les constituants et on chauffe le mélange jusqu'à homogénéité puis on laisse refroidir jusqu'à température ambiante.

### 2. Réalisation de l'émulsion :

On introduit l'une après l'autre les phases A1, A2 et A3 sous agitation Moritz en prenant soin de bien homogénéiser après addition de chacune des phases et d'augmenter la vitesse d'agitation si nécessaire.
On ajoute ensuite la phase A4 sous agitation puis la phase aqueuse B en augmentant la vitesse d'agitation si nécessaire.
On ajoute ensuite successivement les phases C et D et on laisse encore sous agitation pendant 10 minutes.

Les fonds de teint selon l'invention ainsi obtenus (exemples 1, 2, 5, 6 et 7) ont une texture fluide et douce qui s'applique facilement. Le maquillage obtenu unifie le teint tout en restant naturel et sans épaisseur, il laisse sur le visage un dépôt confortable, doux au toucher et mat. Le maquillage présente une bonne tenue dans le temps de sa couleur, de son homogénéité, et sa matité. Il est résistant au transfert.

### Mesure du non transfert sur les compositions des exemples 1 à 4 :

On a comparé la résistance au transfert des compositions des exemples 1 et 2 (selon l'invention), par rapport aux compositions 3 et 4 (hors invention) sur un panel de 10 femmes.
Pour chaque sujet, on nettoie la zone du cou avec un démaquillant non gras (du type Plénitude Refreshing Eye Makeup Remover) et on laisse sécher à l'air pendant 5 minutes.

On applique, avec une éponge, de 0,10 à 0,15 ml de composition sur les deux cotés du cou depuis la ligne délimitée par la mâchoire jusqu'au bas du cou.
On laisse le produit sécher pendant 10 minutes.

On positionne un tissu de 24x33 cm (type tissu Testfabrics Inc., Ref. 100% Bleached Double-Knit Interlock Cotton) le long de la minerve cervicale (type Soft Foam Cervical Collar, OTC Professional Appliances) et on l'attache avec des épingles de sûreté.

On place la minerve autour du cou du sujet pendant 30 minutes.

On enlève alors le tissu et on note visuellement la quantité de produit déposé sur le tissu en utilisant l'échelle suivante :
1= aucune
2= légère
3= modérée
4= importante

L'indice de transfert correspond à la moyenne des notes obtenues sur les 10 sujets. Plus la valeur de cet indice est faible et moins le produit transfère.

### Résultats :

| | Ex 1 (Invention) | Ex 2 (invention) | Ex 3 (comparaison) | Ex 4 (comparaison) |
|---|---|---|---|---|
| Y (polymère vinylique avec motif dendrimère carbosiloxane) | 7,5 | 12,5 | 0 | 12,5 |
| Z (copolymère d'oléfine) | 1,5 | 1,5 | 1,5 | 0 |
| Indice de transfert | 1,30 | 1,30 | 1,40 | 1,50 |

On peut observer que les fonds de teint selon l'invention (exemples 1 et 2) ont un indice de transfert moins élevé que les fonds de teint qui contiennent seulement le polymère vinylique avec motif dendrimère carbosiloxane (exemple 4) ou seulement le copolymère d'oléfine (exemple 3).
On démontre ici une synergie entre les deux polymères : l'association des deux polymères permet d'obtenir une résistance au transfert améliorée.

### Exemples 8 à 12 :

On a préparé 5 fonds de teint ayant les compositions suivantes :

| | | Exemple 8 %massique | Exemple 9 %massique | Exemple 10 %massique | Exemple 11 %massique | Exemple 12 %massique |
|---|---|---|---|---|---|---|
| A1 | Polydiméthylesiloxane alpha-omega oxyéthyléné/ oxypropyléné dans cyclopentasiloxane vendu sous la dénomination Abil EM 97 par la Société Goldschmidt | 1,8 | 1,8 | 1,8 | 1,8 | 1,8 |
| | Isostearyl diglyceryl succinate | 0,5 | 0,5 | 0,5 | 0,5 | 0,5 |
| | Butyl paraben | 0,25 | 0,25 | 0,25 | 0,25 | 0,25 |
| | | | | | | |
| A2 | Isododécane | 9,45 | 9,45 | 9,45 | 9,45 | 9,45 |
| | Isohéxadécane | 5 | 5 | 5 | 5 | 5 |
| | Copolymère bloc styrène-éthylène/propylène vendu sous la référence KRATON G1701E par la Société Kraton Polymers | 0,9 | 1,1 | 1,1 | 0,9 | 1,6 |
| A3 | Cyclopentasiloxane | 12,7 | 10 | 9 | 10,7 | 12 |
| | Oxydes de fer enrobés de stearoyl glutamate d'aluminium | 2,83 | 2,83 | 3,27 | 3,35 | 3,96 |
| | Dioxyde de titane enrobé de stearoyl glutamate d'aluminium | 9,17 | 9,17 | 10,73 | 10,65 | 8,04 |
| | | | | | | |
| A4 | Poudre de nylon 12 | 7 | 7 | 6 | 7 | 7 |
| | | | | | | |
| B | Eau déminéralisée | 24,25 | 24,25 | 24,25 | 24,25 | 24,25 |
| | Conservateurs | 0,75 | 0,75 | 0,75 | 0,75 | 0,75 |
| | Sulfate de Magnésium | 0,7 | 0,7 | 0,7 | 0,7 | 0,7 |
| | Glycérol | 3 | 3 | 3 | 3 | 3 |
| | Octane 1-2 diol | 0,3 | 0,3 | 0,3 | 0,3 | 0,3 |
| | | | | | | |
| C | Copolymère vinylique contenant des chaînes latérales silicones dendritiques dans l'isododécane vendu sous la dénomination FA 4002 ID par la société Dow Corning. | 12,5 | 15 | 15 | 12,5 | 12,5 |
| | | | | | | |
| D | Silice pyrogénée hydrophobe | 0,3 | 0,3 | 0,3 | 0,3 | 0,3 |
| | Silicate de magnésium modifié dans l'isododécane vendu sous la dénomination Bentone Gel ISD V par la société Elementis | 8 | 8 | 8 | 8 | 8 |
| | Total | 100 | 100 | 100 | 100 | 100 |

Le mode de préparation est le même que pour les exemples 1 à 7.

Les fonds de teint ainsi obtenus ont une texture fluide et douce qui s'applique facilement. Le maquillage obtenu unifie le teint tout en restant naturel et sans épaisseur, il laisse sur le visage un dépôt confortable, doux au toucher et mat. Le maquillage présente une bonne tenue dans le temps de sa couleur, de son homogénéité, et sa matité. Il est résistant au transfert.

## Revendications

1. Composition de maquillage ou de soin des matières kératiniques comprenant une phase grasse liquide comprenant :
i) au moins un polymère vinylique ayant au moins un motif dérivé de dendrimère carbosiloxane présent en une teneur en matière active allant de 1 à 10% en poids par rapport au poids total de la composition et,
ii) au moins un copolymère d'oléfine,
**caractérisée en ce que** le polymère vinylique greffé avec un dendrimère carbosiloxane est le produit de polymérisation :
(A) de 0,1 à 99 partie en poids d'un ou plusieurs monomère(s) acrylate ou méthacrylate; et
(B) de 100 à 0,1 parties en poids d'un monomère acrylate ou méthacrylate d'un dendrimère carbosiloxane tri[tri(triméthylsiloxy)silyléthyl diméthylsiloxy]silylpropyle répondant à l'une des formules :

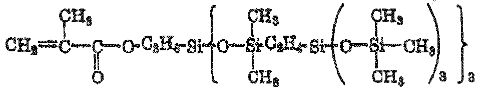

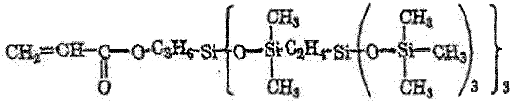

2. Composition selon la revendication 1, **caractérisée en ce que** le polymère vinylique ayant au moins un motif dérivé de dendrimère carbosiloxane est présent en une teneur en matière active allant de de 2 à 7 % en poids par rapport au poids total de la composition.

3. Composition selon l'une quelconque des revendications précédentes, **caractérisée en ce que** le copolymère d'oléfine est choisi parmi les copolymères d'oléfine amorphes ou à cristallisation contrôlée et modérée.

4. Composition selon la revendication précédente, **caractérisée en ce que** le copolymère d'oléfine amorphe est un copolymère bloc choisi parmi les copolymères dibloc, tribloc, multibloc, radial, étoile, et leurs mélanges.

5. Composition selon la revendication précédente, **caractérisée par le fait que** le copolymère bloc est un copolymère bloc hydrocarboné formé par polymérisation de monomères de carbure éthylénique, ayant notamment une ou deux insaturation éthyléniques, ayant de 2 à 5 atomes de carbone.

6. Composition selon la revendication précédente, **caractérisée par le fait que** le copolymère bloc hydrocarboné est formé par polymérisation d'oléfine choisie parmi l'éthylène, le propylène, le butadiène, l'isoprène.

7. Composition selon l'une des revendications 5 ou 6, **caractérisée par le fait que** le copolymère bloc hydrocarboné est un copolymère, éventuellement hydrogéné, à blocs styrène et à blocs éthylène/alkylène en C3-C4.

8. Composition selon l'une quelconque des revendications 5 à 7, **caractérisée par le fait que** le copolymère bloc hydrocarboné est choisi parmi les copolymères dibloc, éventuellement hydrogénés, de styrène-éthylène/propylène, de styrène-éthylène/butadiène, et les copolymères tribloc, éventuellement hydrogénés, de styrène-éthylène/butadiène-styrène, de styrène-isoprène-styrène, et de styrène-butadiène-styrène.

9. Composition selon l'une quelconque des revendications 5 à 8, **caractérisée par le fait que** le copolymère bloc hydrocarboné est un copolymère de styrène-éthylène/propylène.

10. Composition selon la revendication 3, **caractérisé en ce que** le copolymère d'oléfine est un copolymère d'oléfine à cristallisation contrôlée et modérée choisi parmi :
- les copolymères d'α-oléfine, en particulier d'α-oléfine en C₂-C₁₆ et mieux en C₂-C₁₂,
- les copolymères d'éthylène ou de propylène et d'une cyclooléfine, et
- les copolymères de monooléfine et de monomère à liaison(s) éthylénique(s).

11. Composition selon la revendication précédente, **caractérisée en ce que** le copolymère d'oléfine à cristallisation contrôlée et modérée est choisi parmi les copolymères d'éthylène/octène et les copolymères éthylène/norbornène.

12. Composition selon l'une quelconque des revendications précédentes, **caractérisée en ce que** le copolymère d'oléfine est présent dans la composition en une teneur allant de 0,1 % à 10 % en poids, par rapport au poids total de la composition, de préférence allant de 0,2 % à 5 % en poids, et plus préférentiellement allant de 0,5 % à 3 % en poids.

13. Composition selon la revendication 1, **caractérisée en ce que** le copolymère d'oléfine est choisi parmi les copolymères d'oléfine amorphes de styrène-éthylène/propylène.

14. Composition selon l'une quelconque des revendications précédentes, **caractérisée en ce que** la phase grasse liquide comprend au moins une huile.

15. Composition selon la revendication précédente, **caractérisée en ce que** l'huile est une huile volatile.

16. Composition selon l'une quelconque des revendications précédentes, **caractérisée en ce que** la phase grasse liquide comprend deux huiles volatiles différentes.

17. Composition selon les revendications 15 à 16, **caractérisée en ce que** l'huile volatile est présente dans la composition en une teneur allant de 1 à 80 % en poids par rapport au poids total de la composition, de préférence de 5 à 70 % en poids, plus préférentiellement allant de 10 à 60 % en poids, et plus préférentiellement encore de 15 à 50% en poids.

18. Composition selon l'une quelconque des revendications précédentes, **caractérisée en ce qu'**elle comprend un agent épaississant polymérique des huiles choisi parmi :
- les polycondensats de type polyamide résultant de la condensation entre (α) au moins un acide choisi parmi les acides dicarboxyliques comprenant au moins 32 atomes de carbone et (β) un alkylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés ;
- les polymères siliconés du type :
1) des polyorganosiloxanes comportant au moins deux groupes capables d'établir des interactions hydrogène, ces deux groupes étant situés dans la chaîne du polymère, et/ou
2) des polyorganosiloxanes comportant au moins deux groupes capables d'établir des interactions hydrogène, ces deux groupes étant situés sur des greffons ou ramifications,
les groupes capables d'établir des interactions hydrogène pouvant être choisis parmi les groupes ester, amide, sulfonamide, carbamate, thiocarbamate, urée, uréthane, thiourée, oxamido, guanidino, biguanidino et leurs combinaisons.

19. Composition selon l'une quelconque des revendications précédentes, **caractérisée en ce qu'**elle comprend un agent épaississant minéral des huiles choisi parmi les argiles organophiles et les silices pyrogénées.

20. Composition selon l'une quelconque des revendications précédentes, **caractérisé en ce que** la composition comprend au moins une phase pulvérulente choisie parmi les pigments, les charges et/ou les nacres et leurs mélanges.

21. Composition selon la revendication précédente, **caractérisée en ce que** la phase pulvérulente comprend au moins une poudre de polytétrafluoroéthylène.

22. Composition selon l'une quelconque des revendications précédentes, **caractérisé en ce que** la composition comprend un ingrédient cosmétique choisi parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les sequestrants, les agents déodorants, les agents filmogènes, et leurs mélanges.

23. Composition selon l'une quelconque des revendications précédentes, **caractérisé en ce que** ladite composition se présente sous forme anhydre ou sous forme d'émulsion, préférentiellement eau-dans-huile.

24. Procédé non thérapeutique de maquillage ou de soin des matières kératiniques comprenant l'application sur lesdites matières kératiniques d'une composition selon l'une quelconque des revendications 1 à 23.

25. Utilisation d'une composition selon l'une quelconque des revendications 1 à 23, pour obtenir un maquillage sur les matières kératiniques, présentant une bonne tenue de la couleur et de la matité et résistant au transfert.

## Patentansprüche

1. Zusammensetzung zum Schminken oder Pflegen von Keratinstoffen, die eine flüssige Fettphase umfasst, welche Folgendes umfasst:
i) mindestens ein Vinylpolymer mit mindestens einem Baustein, der sich von Carbosiloxan-Dendrimeren ableitet und zu einem Wirkstoffgehalt im Bereich von 1 bis 10 Gewichts-% vorliegt, bezogen auf das Gesamtgewicht der Zusammensetzung, und
ii)mindestens ein Olefincopolymer,
**dadurch gekennzeichnet, dass** das Vinylpolymer, welchem ein Carbosiloxan-Dendrimer aufgepfropft wurde, bei der Polymerisation der folgenden Stoffe entsteht:
(A) 0,1 bis 99 Gewichtsteile eines oder mehrerer Acrylat- oder Methacrylatmonomere; und
(B) 100 bis 0,1 Gewichtsteile eines Acrylat- oder Methacrylatmonomers eines Carbosiloxan-Dendrimers des Typs Tri[tri(trimethylsiloxy)silylethyl dimethylsiloxy]silylpropyl, welches einer der folgenden Formeln entspricht:

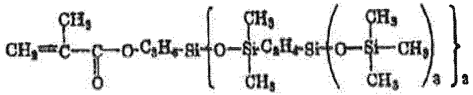

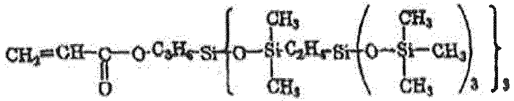

2. Zusammensetzung nach Anspruch 1, **dadurch gekennzeichnet, dass** das Vinylpolymer mit mindestens einem Baustein, der sich von Carbosiloxan-Dendrimeren ableitet, zu einem Wirkstoffgehalt im Bereich von 2 bis 7 Gewichts-% vorliegt, bezogen auf das Gesamtgewicht der Zusammensetzung.

3. Zusammensetzung nach einem beliebigen der vorhergehenden Ansprüche, **dadurch gekennzeichnet, dass** das Olefincopolymer aus den Olefincopolymeren ausgewählt ist, welche amorph sind oder eine gesteuerte und mäßige Kristallisation erfahren haben.

4. Zusammensetzung nach dem vorhergehenden Anspruch, **dadurch gekennzeichnet, dass** es sich bei dem amorphen Olefincopolymer um ein Blockcopolymer handelt, welches aus den Diblock-, Triblock-, Multiblockcopolymeren, den radialen und den Sterncopolymeren sowie deren Mischungen ausgewählt ist.

5. Zusammensetzung nach dem vorhergehenden Anspruch, **dadurch gekennzeichnet, dass** es sich bei dem Blockcopolymer um ein Kohlenwasserstoff-Blockcopolymer handelt, welches bei der Polymerisation von Alkenmonomeren entsteht, die insbesondere eine oder zwei ethylenisch ungesättigte Stellen aufweisen, wobei sie 2 bis 5 Kohlenstoffatome haben.

6. Zusammensetzung nach dem vorhergehenden Anspruch, **dadurch gekennzeichnet, dass** das Kohlenwasserstoff-Blockcopolymer durch Polymerisation von Olefinen gebildet wird, welche aus Ethylen, Propylen, Butadien, Isopren ausgewählt sind.

7. Zusammensetzung nach einem der Ansprüche 5 oder 6, **dadurch gekennzeichnet, dass** es sich bei dem Kohlenwasserstoff-Blockcopolymer um ein möglicherweise hydriertes Copolymer aus Styrolblöcken und Ethylen-/C₃-C₄-Alkylenblöcken handelt.

8. Zusammensetzung nach einem beliebigen der Ansprüche 5 bis 7, **dadurch gekennzeichnet, dass** das Kohlenwasserstoff-Blockcopolymer aus den möglicherweise hydrierten Diblockcopolymeren von Styrol-Ethylen/Propylen, von Styrol-Ethylen/Butadien, und den möglicherweise hydrierten Triblockcopolymeren von Styrol-Ethylen/Butadien-Styrol, von Styrol-Isopren-Styrol und von Styrol-Butadien-Styrol ausgewählt ist.

9. Zusammensetzung nach einem beliebigen der Ansprüche 5 bis 8, **dadurch gekennzeichnet, dass** es sich bei dem Kohlenwasserstoff-Blockcopolymer um ein Copolymer von Styrol/Ethylen/Propylen handelt.

10. Zusammensetzung nach Anspruch 3, **dadurch gekennzeichnet, dass** es sich bei dem Olefincopolymer um ein Olefincopolymer mit gesteuerter und mäßiger Kristallisation handelt, welches aus den folgenden ausgewählt ist:
- den Copolymeren von α-Olefin, insbesondere von C₂-C₁₆- und besser noch von C₂-C₁₂-α-Olefin,
- den Copolymeren von Ethylen oder von Propylen und von einem Cycloolefin, und
- den Copolymeren von Monoolefin und von Monomeren mit ethylenischer/ethylenischen Bindung(en).

11. Zusammensetzung nach dem vorhergehenden Anspruch, **dadurch gekennzeichnet, dass** das Olefincopolymer mit gesteuerter und mäßiger Kristallisation aus den Ethylen/Octen-Copolymeren und den Ethylen/Norbornen-Copolymeren ausgewählt ist.

12. Zusammensetzung nach einem beliebigen der vorhergehenden Ansprüche, **dadurch gekennzeichnet, dass** das Olefincopolymer in der Zusammensetzung zu einem Gehalt im Bereich von 0,1 bis 10 Gewichts-%, bezogen auf das Gesamtgewicht der Zusammensetzung, vorzugsweise im Bereich von 0,2 bis 5 Gewichts-%, und stärker bevorzugt von 0,5 bis 3 Gewichts-% vorliegt.

13. Zusammensetzung nach Anspruch 1, **dadurch gekennzeichnet, dass** das Olefincopolymer aus den amorphen Olefincopolymeren von Styrol-Ethylen/Propylen ausgewählt ist.

14. Zusammensetzung nach einem beliebigen der vorhergehenden Ansprüche, **dadurch gekennzeichnet, dass** die flüssige Fettphase mindestens ein Öl umfasst.

15. Zusammensetzung nach dem vorhergehenden Anspruch, **dadurch gekennzeichnet, dass** es sich bei dem Öl um ein flüchtiges Öl handelt.

16. Zusammensetzung nach einem beliebigen der vorhergehenden Ansprüche, **dadurch gekennzeichnet, dass** die flüssige Fettphase zwei verschiedenartige flüchtige Öle umfasst.

17. Zusammensetzung nach den Ansprüchen 15 bis 16, **dadurch gekennzeichnet, dass** das flüchtige Öl in der Zusammensetzung zu einem Gehalt im Bereich von 1 bis 80 Gewichts-%, bezogen auf das Gesamtgewicht der Zusammensetzung, vorzugsweise von 5 bis 70 Gewichts-%, stärker bevorzugt von 10 bis 60 Gewichts-%, und wiederum stärker bevorzugt von 15 bis 50 Gewichts-%, vorliegt.

18. Zusammensetzung nach einem beliebigen der vorhergehenden Ansprüche, **dadurch gekennzeichnet, dass** sie ein polymerartiges Mittel zum Verdicken der Öle umfasst, welches aus den folgenden ausgewählt ist:
- den Polykondensationsprodukten vom Typ Polyamid, welche bei der Kondensation zwischen (α) mindestens einer Säure, die aus den Dicarbonsäuren ausgewählt ist, welche mindestens 32 Kohlenstoffatome umfassen, und (β) einem Alkylendiamin entstehen, wobei das Polyamidpolymer mindestens eine endständige Carbonsäuregruppe umfasst, die mit mindestens einem Monoalkohol oder einem Monoamin, welcher/welches 12 bis 30 geradkettige und gesättigte Kohlenstoffatome umfasst, verestert beziehungsweise in ein Amid überführt wurde;
- den Silikonpolymeren des Typs:
1) Polyorganosiloxane, die mindestens zwei Gruppen aufweisen, welche dazu befähigt sind, Wasserstoffwechselwirkungen herzustellen, wobei diese beiden Gruppen sich in der Kette des Polymers befinden, und/oder
2) Polyorganosiloxane, die mindestens zwei Gruppen aufweisen, welche dazu befähigt sind, Wasserstoffwechselwirkungen herzustellen, wobei diese beiden Gruppen sich an den aufgepfropften Seitenketten oder an den Verzweigungen befinden,
wobei die Gruppen, welche dazu befähigt sind, Wasserstoffwechselwirkungen herzustellen, aus den Ester-, Amid-, Sulfonamid-, Carbamat-, Thiocarbamat-, Harnstoff-, Urethan-, Thioharnstoff-, Oxamido-, Guanidino-, Biguanidinogruppen und deren Kombinationen ausgewählt sind.

19. Zusammensetzung nach einem beliebigen der vorhergehenden Ansprüche, **dadurch gekennzeichnet, dass** sie ein mineralisches Mittel zum Verdicken der Öle umfasst, welches aus den organophilen Tonen und den pyrogenen Siliziumdioxiden ausgewählt ist.

20. Zusammensetzung nach einem beliebigen der vorhergehenden Ansprüche, **dadurch gekennzeichnet, dass** die Zusammensetzung mindestens eine pulverförmige Phase umfasst, welche aus den Pigmenten, den Füllstoffen und/oder den Perlglanzmitteln und deren Mischungen ausgewählt ist.

21. Zusammensetzung nach dem vorhergehenden Anspruch, **dadurch gekennzeichnet, dass** die pulverförmige Phase mindestens ein Polytetrafluorethylenpulver umfasst.

22. Zusammensetzung nach einem beliebigen der vorhergehenden Ansprüche, **dadurch gekennzeichnet, dass** die Zusammensetzung einen kosmetischen Inhaltsstoff umfasst, der aus den Antioxidantien, den Duftstoffen, den Konservierungsstoffen, den Neutralisierungsmitteln, den Tensiden, den Sonnenschutzfiltern, den Vitaminen, den feuchtigkeitsspendenden Mitteln, den Selbstbräunern, den hautglättenden Mitteln, den erweichenden Mitteln, den hydrophilen oder lipophilen Wirkstoffen, den Mitteln gegen freie Radikale, den Komplexierungsmitteln, den desodorierenden Mitteln, den filmbildenden Mitteln und deren Mischungen ausgewählt ist.

23. Zusammensetzung nach einem beliebigen der vorhergehenden Ansprüche, **dadurch gekennzeichnet, dass** die Zusammensetzung in wasserfreier Form oder in Form einer Emulsion, vorzugsweise Wasser-in-Öl, vorliegt.

24. Nicht-therapeutisches Verfahren zum Schminken oder Pflegen von Keratinstoffen, welches das Aufbringen einer Zusammensetzung nach einem beliebigen der Ansprüche 1 bis 23 auf die Keratinstoffe umfasst.

25. Verwendung einer Zusammensetzung nach einem beliebigen der Ansprüche 1 bis 23, um bei den Keratinstoffen eine Schminkwirkung zu erzielen, die eine gute Beständigkeit des Farbtons und des Mattgrads aufweist und übertragungsfest ist.

## Claims

1. Composition for making up or caring for keratin materials, comprising a liquid fatty phase comprising:
i) at least one vinyl polymer having at least one carbosiloxane dendrimer-based unit present in an amount of active material ranging from 1% to 10% by weight relative to the total weight of the composition, and
ii) at least one olefin copolymer,
**characterized in that** the vinyl polymer grafted with a carbosiloxane dendrimer is the product of polymerization:
(A) of 0.1 to 99 parts by weight of one or more acrylate or methacrylate monomer(s) ; and
(B) of 100 to 0.1 parts by weight of an acrylate or methacrylate monomer of a tri[tri(trimethylsiloxy)silylethyldimethylsiloxy]silylp ropyl carbosiloxane dendrimer corresponding to one of the formulae:

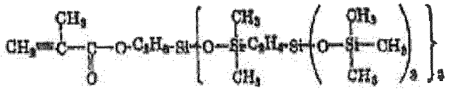

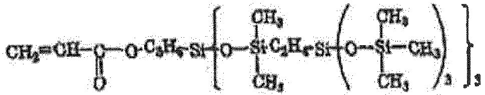

2. Composition according to Claim 1, **characterized in that** the vinyl polymer having at least one carbosiloxane dendrimer-based unit is present in a content of active material ranging from 2% to 7% by weight relative to the total weight of the composition.

3. Composition according to either one of the preceding claims, **characterized in that** the olefin copolymer is chosen from amorphous olefin copolymers or olefin copolymers with controlled and moderate crystallization.

4. Composition according to the preceding claim, **characterized in that** the amorphous olefin copolymer is a block copolymer chosen from diblock, triblock, multiblock, radial and star copolymers, and blends thereof.

5. Composition according to the preceding claim, **characterized in that** the block copolymer is a hydrocarbon-based block copolymer formed by polymerization of ethylenic carbide monomers, having in particular one or two ethylenic unsaturations and having from 2 to 5 carbon atoms.

6. Composition according to the preceding claim, **characterized in that** the hydrocarbon-based block copolymer is formed by polymerization of an olefin chosen from ethylene, propylene, butadiene and isoprene.

7. Composition according to either of Claims 5 and 6, **characterized in that** the hydrocarbon-based block copolymer is an optionally hydrogenated copolymer comprising styrene blocks and ethylene/C₃-C₄ alkylene blocks.

8. Composition according to any one of Claims 5 to 7, **characterized in that** the hydrocarbon-based block copolymer is chosen from optionally hydrogenated styrene-ethylene/propylene and styrene-ethylene/butadiene diblock copolymers and optionally hydrogenated styrene-ethylene/butadiene-styrene, styrene-isoprene-styrene and styrene-butadiene-styrene triblock copolymers.

9. Composition according to any one of Claims 5 to 8, **characterized in that** the hydrocarbon-based block copolymer is a styrene-ethylene/propylene copolymer.

10. Composition according to Claim 3, **characterized in that** the olefin copolymer is an olefin copolymer having controlled and moderate crystallization, chosen from:
- α-olefin copolymers, in particular C₂-C₁₆ and better still C₂-C₁₂ α-olefin copolymers,
- copolymers of ethylene or propylene and of a cycloolefin, and
- copolymers of monoolefin and of a monomer comprising an ethylenic bond or bonds.

11. Composition according to the preceding claim, **characterized in that** the olefin copolymer having controlled and moderate crystallization is chosen from ethylene/octene copolymers and ethylene/norbornene copolymers.

12. Composition according to any one of the preceding claims, **characterized in that** the olefin copolymer is present in the composition in a content ranging from 0.1% to 10% by weight, relative to the total weight of the composition, preferably ranging from 0.2% to 5% by weight, and more preferentially ranging from 0.5% to 3% by weight.

13. Composition according to Claim 1, **characterized in that** the olefin copolymer is chosen from amorphous olefin copolymers of styrene-ethylene/propylene.

14. Composition according to any one of the preceding claims, **characterized in that** the liquid fatty phase comprises at least one oil.

15. Composition according to the preceding claim, **characterized in that** the oil is a volatile oil.

16. Composition according to any one of the preceding claims, **characterized in that** the liquid fatty phase comprises two different volatile oils.

17. Composition according to Claims 15 and 16, **characterized in that** the volatile oil is present in the composition in a content ranging from 1% to 80% by weight relative to the total weight of the composition, preferably from 5% to 70% by weight, more preferentially ranging from 10% to 60% by weight, and even more preferentially from 15% to 50% by weight.

18. Composition according to any one of the preceding claims, **characterized in that** it comprises a polymeric thickener of oils, chosen from:
- polycondensates of polyamide type resulting from the condensation between (α) at least one acid chosen from dicarboxylic acids comprising at least 32 carbon atoms and (β) an alkylenediamine, in which the polyamide polymer comprises at least one end carboxylic acid group esterified or amidated with at least one monoalcohol or one monoamine comprising from 12 to 30 carbon atoms, which are linear and saturated;
- silicone polymers of the type such as:
1) polyorganosiloxanes comprising at least two groups capable of establishing hydrogen interactions, these two groups being located in the polymer chain, and/or
2) polyorganosiloxanes comprising at least two groups capable of establishing hydrogen interactions, these two groups being located on grafts or branches,
the groups capable of establishing hydrogen interactions possibly being chosen from ester, amide, sulphonamide, carbamate, thiocarbamate, urea, urethane, thiourea, oxamido, guanidino and biguanidino groups, and combinations thereof.

19. Composition according to any one of the preceding claims, **characterized in that** it comprises a mineral thickener of oils, chosen from organophilic clays and fumed silicas.

20. Composition according to any one of the preceding claims, **characterized in that** the composition comprises at least one pulverulent phase chosen from pigments, fillers and/or nacres, and mixtures thereof.

21. Composition according to the preceding claim, **characterized in that** the pulverulent phase comprises at least one polytetrafluoroethylene powder.

22. Composition according to any one of the preceding claims, **characterized in that** the composition comprises a cosmetic ingredient chosen from antioxidants, fragrances, preservatives, neutralizing agents, surfactants, sunscreens, vitamins, moisturizing agents, self-tanning compounds, antiwrinkle active agents, emollients, hydrophilic or lipophilic active agents, free-radical scavengers, sequestering agents, deodorant agents, film-forming agents, and mixtures thereof.

23. Composition according to any one of the preceding claims, **characterized in that** said composition is in anhydrous form or in the form of an emulsion, preferentially a water-in-oil emulsion.

24. Non-therapeutic process for making up or caring for keratin materials, comprising the application, to said keratin materials, of a composition according to any one of Claims 1 to 23.

25. Use of a composition according to any one of Claims 1 to 23, for obtaining a makeup result on keratin materials which has good colour and mattness wear properties and is transfer resistant.
